# EUROPEAN PATENT APPLICATION

(11) **EP 1 314 780 A1**
(43) Date of publication of application: **28.05.2003**
(21) Application number: 01956898.9
(22) Date of filing: 15.08.2001
(51) Int. Cl.: C12N 15/09, C07K 14/78, C07K 19/00, C12N 1/21, C12N 5/10, A61K 38/00

(54) **COLLAGEN-BINDING HYBRID POLYPEPTIDE**

(30) Priority: 15.08.2000 JP 2000246341
(71) Applicant: TERUMO KABUSHIKI KAISHA, Tokyo 151-0072 (JP)
(72) Inventor: ISHIKAWA, Tetsuya, Terumo kabushiki kaisha, Ashigarakami-gun, Kanagawa25 9-0151 (JP); KITAJIMA, Takashi, Terumo kabushiki kaisha, Ashigarakami-gun, Kanagawa 2559-0151 (JP)
(74) Representative: Casalonga, Axel
(86) International application number: JP0107036
(87) International publication number: WO02014505

(57) **Abstract**

A collagen-binding hybrid polypeptide wherein the collagen-binding ability is imparted to a functional polypeptide is provided.

The hybrid polypeptide is obtained by ligating a collagen-binding domain having an amino acid sequence which constitutes the polypeptide in human fibronectin from Ala ²⁶⁰ to Arg ⁴⁸⁴ to the functional polypeptide by a genetic engineering technique.

The collagen-binding hybrid polypeptide is useful in a drug delivery system (DDS) for the functional polypeptide. Moreover, this polypeptide can be formulated into a complex together with collagen. The functionally modified collagen matrix thus obtained is useful as a new biomaterial for tissue regeneration.

## Description

### Technical Field

This invention relates to a hybrid polypeptide, and more specifically, to a hybrid polypeptide which is produced by connecting a collagen-binding domain having an amino acid sequence in human fibronectin from Ala ²⁶⁰ to Arg ⁴⁸⁴ to a functional polypeptide by a genetic engineering technique and a biomaterial produced by combining the hybrid polypeptide with a polypeptide from collagen.

### Background Art

Cytokines, growth factors and other functional polypeptides are expected for their use as a pharmaceutical. However, functional polypeptides generally suffer from insufficient stability in living tissues. Functional polypeptides also suffer from the difficulty in realizing topical retention and sustained release in target tissues, and as a consequence, likeliness of inducing side effects in other parts of the body. In view of such situation, practical use of many of such functional polypeptides is deemed difficult, and there is a strong demand for an efficient drug delivery system (DDS) of such functional polypeptides.

Collagen has been expected as a promising carrier capable of improving the sustained release property of a functional polypeptide. Affinity of the functional polypeptide for collagen, however, is generally low, and it has been so far impossible to retain the functional polypeptide in the collagen matrix for prolonged period in a stable manner by merely mixing the polypeptide with the collagen.

The approach of chemical cross-linking of the functional polypeptide with the collagen has also suffered from the problem of loss or reduction of the activity.

In the more recent techniques developed in view of such situation, the functional polypeptide is connected with other polypeptides by means of genetic engineering to thereby realize targeting of the functional polypeptide. To be more specific, expression of a hybrid polypeptide in Escherichia coli (*E. coli)* or the like has been enabled by the genetic recombination technology. The expressed hybrid polypeptide, however, does not necessarily exhibit its original activity, and it is difficult to maintain the activity.

JP 5-178897 A discloses a method for producing a functional polypeptide by ligating bFGF with the cell-binding domain sequence of fibronectin (FN), which is a component of extracellular matrix. To be more specific, there is disclosed a hybrid polypeptide wherein a polypeptide having 277 amino acid residues in human FN from Pro ¹²³⁹ to Ser ¹⁵¹⁵ is ligated with bFGF. The polypeptide having the amino acid residues in human FN from Pro 1239 to Ser ¹⁵¹⁵ consists of an amino acid sequence which is quite different from that of the polypeptide included on the N terminal side of human FN from about 28 kDa to about 75 kDa in which the collagen-binding domain is located, and has no collagen-binding ability. This hybrid polypeptide binds to cells to exhibit the cell-growth promoting activity. However, long-term sustained release or topical retention is not realized due to the direct binding of the polypeptide to the cells. The inventors of JP 5-178897 A also reported significantly reduced bFGF activity of the functional polypeptide (Hashi H., et al., "Angiogenic Activity of a Fusion Protein of the Cell-Binding Domain of Fibronectin and Basic Fibroblast Growth Factor" CELL STRUCTURE AND FUNCTION 19:37-47(1994)).

Another attempt for realizing the-sustained release through the extracellular matrix is collagen targeting TGF-β which is a hybrid polypeptide of a collagen-binding decapeptide from bovine von Willebrand factor and transforming growth factor β (TGF-β) (Tuan et al., Connective Tissue Research, vol. 34, No. 1, pages 1 to 9 (1996), Han et al., Protein Expression and Purification, vol. 11, pages 169 to 178 (1997), and Gordon et al., Human Gene Therapy, vol. 8, pages 1385 to 1394 (1997)).

Further, US 5,800,811 discloses a hybrid polypeptide of a collagen-binding decapeptide from bovine von Willebrand factor and TGF-β, and WO 00/06195 discloses a hybrid polypeptide of a collagen-binding decapeptide from bovine von Willebrand factor and vascular endothelial growth factor (VEGF).

In addition, Nishi et al. produced a collagen-binding cell growth factor which was a hybrid polypeptide of bFGF or EGF with a collagen-binding polypeptide from collagenase *of Clostridium histolyticum,* which is an anaerobic Gram-positive bacillus (Proc. Natl. Acad. Sci. USA, vol. 95, pages 7018 to 7023 (1998)).

The collagen-binding domain of FN may be used as a collagen (gelatin)-binding polypeptide partner. That is, use of a polypeptide from FN as a tag in the purification of a desired protein is proposed. To be more specific, a vector capable of expressing a polypeptide including the gelatin-binding domain of FN connected to the desired protein, and a method of purifying the desired protein are proposed in USP 5,342,762 and USP 5,460,955, and a polypeptide comprising the collagen-binding domain of FN connected to other polypeptide or a therapeutic agent, and its purification method are proposed in JP 62-89699 A.

JP 8-140677 A discloses another technique for targeting an active protein by insolubilizing it in the extracellular matrix. In this technique, a chimera protein is produced, and in this protein, an amino acid sequence from the exogenous protein or polypeptide is inserted between 70 kDa fragment at the N terminal of the FN molecule and 37 kDa fragment at the C terminal of the FN molecule.

It should be noted that the conventional investigations as described above have failed to provide a hybrid polypeptide which includes a collagen-binding polypeptide and a functional polypeptide and which has both the collagen-binding ability and the activity of the functional polypeptide. In practical point of view, it is important to exhibit the activity of the functional polypeptide even after binding to collagen, which has not been demonstrated by the conventional hybrid polypeptides.

### Disclosure of the Invention

An object of the present invention is to provide a collagen-binding hybrid polypeptide wherein the activity of the functional polypeptide is maintained and the collagen-binding ability is imparted.

In order to solve the problems as described above, it has been found that a collagen-binding hybrid polypeptide wherein the activity of a functional polypeptide is maintained and the collagen-binding ability is imparted can be produced by ligating a collagen-binding domain which has an amino acid sequence constituting the polypeptide in human fibronectin from Ala ²⁶⁰ to Arg ⁴⁸⁴ with the functional polypeptide by a genetic engineering technique. It has also been found that a biomaterial (a functional polypeptide/collagen composite) can be produced by combining the collagen-binding hybrid polypeptide with a polypeptide from collagen. The present invention has been accomplished on the basis of such findings. That is, the objects of the present invention are achieved by 1) to 15) as described below.
1) A hybrid polypeptide wherein a functional polypeptide is ligated to a collagen-binding domain which has an amino acid sequence constituting the polypeptide in human fibronectin from Ala ²⁶⁰ to Arg ⁴⁸⁴; an amino acid sequence which is homologous to said amino acid sequence; or an amino acid sequence wherein one or more amino acid residues are deleted, substituted, inserted, or added in said amino acid sequence.
   The term "collagen-binding ability" used herein is the same as the "gelatin-binding ability".
2) A hybrid polypeptide according to the above 1) wherein said functional polypeptide is ligated to the carboxyl terminal of the collagen-binding domain.
3) A hybrid polypeptide according to the above 1) or 2) wherein the functional polypeptide is a cell growth factor or a cytokine.
4) A hybrid polypeptide according to the above 1) to 3) wherein the functional polypeptide is a cell growth factor belonging to the fibroblast growth factor family or epidermal growth factor family.
5) A hybrid polypeptide according to the above 1) to 4) wherein the functional polypeptide is a basic fibroblast growth factor or an epidermal growth factor.
6) A hybrid polypeptide according to the above 1) to 5) wherein the functional polypeptide is ligated to the collagen-binding domain by a genetic engineering process.
7) A hybrid polypeptide according to the above 1) to 6) wherein the functional polypeptide is ligated to the collagen-binding domain by a genetic engineering process, and the hybrid polypeptide is produced in bacteria or yeast, preferably in *E*. *coli.*
   The hybrid polypeptide may have an amino acid or a peptide inserted as a spacer at the ligation site of the functional polypeptide and the collagen-binding domain.
   In such a case, either the spacer, or the spacer and its adjacent sequence may preferably include a protease recognition sequence.
   The protease recognition sequence is preferably a sequence recognizing enterokinase, blood coagulation factor Xa, thrombin, precision (artificially prepared recognition sequence-specific protease), kallikrein, Genenase I, or renin. The protease recognition sequence is further preferably an enterokinase recognition sequence.
8) A hybrid polypeptide according to the above 1) to 7) wherein the hybrid polypeptide is soluble in water.
9) A hybrid polypeptide according to the above 1) to 8) wherein the functional polypeptide after its binding to collagen exhibits its function by being retained in the collagen or by being gradually released from the collagen.
10) An agent for enabling topical retention or sustained release of a functional polypeptide which contains a hybrid polypeptide of any one of the above 1) to 9).
11) A biomaterial containing a functional polypeptide/collagen composite wherein a hybrid polypeptide of any one of the above 1) to 9) is combined with a polypeptide from collagen.
12) A method of promoting or inhibiting proliferation, differentiation, regeneration or biological substance production in a cell, a tissue or an organ by using a biomaterial of the above 11).
13) An agent for enabling topical retention or sustained release of a functional polypeptide which contains a biomaterial of the above (11) or (12).
14) A gene coding for a hybrid polypeptide of any one of the above 1) to 9).
   A recombinant vector including a gene coding for a hybrid polypeptide of any one of the above 1) to 9).
   A transformant including a gene coding for a hybrid polypeptide of any one of the above 1) to 9).
   A bacterium including a gene coding for a hybrid polypeptide of any one of the above 1) to 9).
15) A transformant containing a recombinant vector including a gene coding for a hybrid polypeptide of any one of the above 1) to 9).

The transformant includes bacteria, yeast, insect cell, and animal cell.

A bacterium containing a recombinant vector including a gene coding for a hybrid polypeptide of any one of the above 1) to 9).

*E. coli* containing a recombinant vector including a gene coding for a hybrid polypeptide of any one of the above 1) to 9).

A method for producing a hybrid polypeptide of any one of the above 1) to 9) by using *E. coli.*

### Best Mode for Carrying out the Invention

Fibronectin (hereinafter also referred to as "FN") is a cell-adhesive glycoprotein found in plasma and extracellular matrix as well as on the surface of the cultivated cells. Fibronectin is known to be capable of binding to high molecular weight biomolecules such as collagen (gelatin), heparin, fibrin, and integrin, and to be involved in cell adhesion, tissue organization, repairing of the injured tissue, and other biological processes (Ruoslahti, Annual Review of Biochemistry, vol. 57, pages 375 to 413 (1988)).

The present invention provides a collagen-binding hybrid polypeptide in which a specific collagen-binding domain obtained by protease hydrolysis of fibronectin is ligated to a functional polypeptide, and the polypeptide of the present invention has both the collagen-binding ability and the activity of the functional polypeptide and the functional polypeptide exhibits its activity after binding to collagen. In other words, the collagen-binding hybrid polypeptide of the present invention is a hybrid polypeptide in which a collagen-binding domain having an amino acid sequence in human fibronectin from Ala ²⁶⁰ to Arg ⁴⁸⁴ is ligated to a functional polypeptide by a genetic engineering process.

It has been found that the collagen-binding ability and the activity of the functional polypeptide can be maintained at a sufficient level by using a specific collagen-binding domain from FN for the collagen-binding polypeptide as described above, and that the activity of the functional polypeptide is also maintained after the binding of the hybrid polypeptide to collagen. On the basis of such findings, the inventors of the present invention invented the collagen-binding hybrid polypeptide which is a novel functional polypeptide as well as its application.

The present invention has also enabled to impart the collagen-binding ability based on the collagen-binding domain to the functional polypeptide which has no collagen-binding ability as well as the functional polypeptide which inherently has the collagen-binding ability.

The term "collagen" used herein includes collagen as well as thermally denatured collagen such as gelatin. Therefore, the term "gelatin-binding" ability is substantially equivalent to "collagen-binding" ability, and in the specification, the collagen-binding ability refers to both the abilities.

The collagen-binding ability of a specific polypeptide derived from FN is quite stable and high in the hybrid polypeptide, and such stable collagen-binding ability has enabled to complete a functional polypeptide/collagen composite which is a novel biomaterial in which, after binding to collagen, the functional polypeptide exhibits its activity while being retained in the collagen or gradually released therefrom.

Furthermore, the specific collagen-binding hybrid polypeptide of the present invention has the nature that its collagen-binding ability is competitively inhibited by the plasma FN. In other words, the collagen-binding hybrid polypeptide of the present invention is released from the collagen when it is exposed to plasma, or in the co-presence or upon addition of the plasma, while it is retained in the collagen by scarcity of the plasma, that is, in a site having no humoral factor (cell growth factor, cytokine or enzyme).

Therefore, it has been critical in completing the present invention that the specific collagen-binding domain derived from FN was used for the collagen-binding polypeptide part in the production of the collagen-binding hybrid polypeptide.

In the state of the recombinant DNA technology and the genetic engineering technology of today, in principle, it is possible to produce a fusion gene with the gene of the functional polypeptide by using the gene coding for the polypeptide from every part of the FN. However, an adequate selection of the collagen-binding domain sequence is critical for the production of a hybrid polypeptide (fusion protein) wherein both the collagen-binding ability and the activity of the functional polypeptide are maintained. Such sequence selection is one of the important features of the present invention.

### <Collagen-binding domain from FN (FNCBD) of the invention>

The collagen-binding domain constituting the present invention has an amino acid sequence constituting a polypeptide obtained by proteolysis of FN using a protease, and preferably, by limited hydrolysis with plasmin and trypsin. The collagen-binding domain has an amino acid sequence which constitutes the polypeptide in human fibronectin from Ala ²⁶⁰ to Arg ⁴⁸⁴; an amino acid sequence which is homologous to the above sequence; or an amino acid sequence in which one or more amino acids and preferably not more than three amino acids have been deleted, substituted, inserted, or added. The collagen-binding domain from FN is hereinafter also referred to as "FNCBD".

Preferably, FN is human FN. Use of FN from other animal species is also acceptable.

In some cases, use of a sequence which is slightly different from the amino acid sequence of the polypeptide in human fibronectin from Ala ²⁶⁰ to Arg ⁴⁸⁴ does not result in the loss of the collagen-binding ability of the resulting hybrid polypeptide.

However, in most cases, the hybrid polypeptide obtained by expressing in *E. coli* a fusion polypeptide of an FN-derived amino acid sequence which can be obtained only by a genetic engineering process with a functional polypeptide utterly disregarding the site cleaved by a protease, exhibits significantly reduced collagen-binding ability even if the FN-derived part used has a sequence covering all or part of the collagen-binding domain.

In view of such situation, when the hybrid collagen-binding hybrid polypeptide is prepared by a genetic engineering process, the sequence of the collagen-binding domain from FN should be prepared by selecting a sequence homologous to the collagen-binding domain which is produced by limited hydrolysis with a protease.

Although considerable number of studies have been conducted on the collagen-binding domain from FN, there are discrepancies between the studies and many points are not elucidated yet. (Owens et al., EMBO J., vol. 5, pages 2825 to 2830 (1986), Ingham et al., J. Biol. Chem, vol. 264, pages 16977 to 16980 (1989), Litvinovich et al., J. Mol. Biol., vol. 217, pages 563 to 575 (1991), Banyai et al., Eur. J. Biochem, vol. 193, pages 801 to 806 (1990), Skorstengaard et al., FEBS letters, vol. 343, pages 47 to 50 (1994), Shimizu et al., Biochimica et Biophysica Acta, vol. 1339, pages 53 to 61 (1997)).

Such discrepancies between the studies are caused by the conclusion drawn without considering the difference between the polypeptide obtained by the genetic engineering technique and the polypeptide obtained by protease cleavage.

Selection of the sequence divided at an inadequate site and addition of purification tag in the production of the FN-derived part from FN by the genetic engineering technique are the causes for the loss or decrease of the collagen-binding ability even if the particular region has the collagen-binding ability inherent in the polypeptide from FN. Particularly in the case of a hybrid polypeptide, the collagen-binding ability of the polypeptide from FN is often lost by the fusion even in the case wherein such activity had been present in the polypeptide from FN before its fusion. That is, it is likely that the original higher order structure of the polypeptides is distorted by the inadequate adoption of the FN-derived polypeptide to invite the loss of activity in the fusion polypeptide.

The collagen-binding hybrid polypeptide of the present invention is produced by the fusion of the functional polypeptide with the collagen-binding domain having the amino acid sequence of the polypeptide in human fibronectin from Ala ²⁶⁰ to Arg ⁴⁸⁴ having no purification tag added thereto. The collagen-binding hybrid polypeptide of the present invention is a recombinant hybrid polypeptide produced in *E. coli* which binds with gelatin sepharose by the collagen-binding ability of FN, is highly adapted for affinity purification and also exhibits highly-maintained activity of the functional polypeptide derived from the functional polypeptide. It should be noted that the present invention is also applicable not only to a system for producing and purifying hybrid polypeptides but also to a system for producing and purifying functional polypeptides in combination with a specific sequence-recognizing protease.

### <Functional polypeptide>

The term "functional polypeptide" used herein is a generic designation of the other polypeptides than those from fibronectin which can be produced by genetic engineering techniques. Examples thereof include cell growth factors including angiogenesis promoters, that is, bFGF family such as basic fibroblast growth factors and acidic fibroblast growth factors, vascular endothelial growth factors (VEGF110. 121, 165, 189, 206, -B, -C, -D), IL-8, IL-4, PD-ECGF, HGF, angiogenin, cell growth factors belonging to EGF family (TGF-α, heparin-binding EGF-like growth factor, EGF, amphiregulin, SDGF, β-cellulin), PDGF, integrinαvβ3, angiopoietin-1. pleiotrophin, midkine, tissue factor, TNF-α (low concentration), IGF, CYR61, PDGF, NK1 and NK2 of HGF, ephrin-B2, matrix metalloproteinase, G-CSF, and growth hormone, angiogenesis inhibitors, that is, endostatin, angiostatin, NK4 of HGF, thrombomodulin, IFN-α/INF-(β, TNF-α (high concentration), TGF-β, IL-1, IL-12, IP-10, GRO-β, PF-4, chondromodulin, angiopoietin-2, TIMP-1,2, chondrocyte-derived inhibiting factor, prothrombin (kringle 2), transforming growth factor β super family (TGF-β, activin, inhibin, follistatin, BMP family, Vg-1, GDF, nodal, dorsalin, MIS, GDNF), epidermal growth factor family (EGF, amphiregulin, SDGF, HB-EGF, β-cellulin, neuregulins, TGF-α), platelet-derived growth factors (PDGF-AA, AB, BB), insulin-like growth factor (IGF), nerve growth factor family (NGF, NT-3, NT-4, NT-6, neurotrophin, BDNF and the like), vascular endothelial growth factor family (VEGF, VEGF-B, -C, -D, PlGF), hepatocyte growth factor (HGF) family, connective tissue growth factor (CTGF) and other cell growth factors, and cell differentiation factors such as bone-morphogenetic protein (BMP) family; cytokines (lymphokine, monokine, chemokine) including interferons (IFN-α, -β, -γ), interleukins (ILs), colony-stimulating factors (GM-CSF, G-CSF, M-CSF), erythropoietin, thrombopoietin, tumor necrosis factors (TNFα, TNFβ), stem cell factor, macrophage migration inhibitory factor, leukemia inhibitory factor; various hormones such as insulin, parathyroid hormone (PTH), growth hormone and glucagon; enzymes such as matrix metalloproteinases (MMPs), streptokinase and superoxide dismutase; natriuretic peptides (ANP, BNP, CNP), platelet factor (PF-4), RANTES, MCP-1, protein C, TFPI, thrombomodulin, antithrombin III, blood coagulation factor-inhibiting proteins, integrilin, Cox-1, TEPI, abciximab (including single-chain antibody), Hirudin, tPA (tissue plasminogen activator), uPA (urokinase), heparinase, and plasmin. In addition, polypeptides which may have any action on a cell, tissue, organ or organism are considered to be functional polypeptides even if the details of their action mechanisms are not elucidated. The functional polypeptides essentially encompass the polypeptides effective in the delivery system in terms of topical retention or targeting.

The functional polypeptide of the present invention may have an amino acid sequence which constitutes the polypeptide as described above, an amino acid sequence which is homologous to this amino acid sequence, or an amino acid sequence in which one or more amino acids are deleted, substituted, inserted or added.

### <Collagen-binding hybrid polypeptide>

In the collagen-binding hybrid polypeptide wherein the collagen-binding domain (FNCBD) having the amino acid sequence which constitutes the polypeptide in human fibronectin from Ala ²⁶⁰ to Arg ⁴⁸⁴ is ligated to the functional polypeptide, the functional polypeptide is desirably ligated on the carboxyl terminal side of the collagen-binding domain. The collagen-binding hybrid polypeptide is preferably soluble in water and can be produced in an industrial manner using bacteria, preferably *E. coli.* Production in yeast, insect cell, or animal cell is also possible.

The hybrid polypeptide of the present invention wherein the collagen-binding domain having the amino acid sequence which constitutes the polypeptide in human fibronectin from Ala ²⁶⁰ to Arg ⁴⁸⁴ is ligated to the functional polypeptide has the following excellent points as compared with the hybrid polypeptide which can be produced in *E. coli* in the same manner and in which the collagen-binding domain having the amino acid sequence which constitutes the polypeptide in human fibronectin from Ala ²⁶⁰ to Trp ⁵⁹⁹ is ligated to the functional polypeptide. To be more specific, since the human fibronectin has a sensitive site to any of the proteases described below between Asp ⁴⁸⁵ and Trp ⁵⁹⁹, in the hybrid polypeptide wherein the collagen-binding domain having the amino acid sequence which constitutes the polypeptide in human fibronectin from Ala ²⁶⁰ to Trp ⁵⁹⁹is ligated to the functional polypeptide, the functional polypeptide is separated from the collagen-binding domain through cleavage in the presence of an excess amount of cathepsin G, cathepsin D, chymotrypsin, thermolysin, leucocyte elastase or chymase, which makes difficult the prolonged topical retention. On the other hand, the hybrid polypeptide of the present invention wherein the collagen-binding domain having the amino acid sequence which constitutes the polypeptide in human fibronectin from Ala ²⁶⁰ to Arg ⁴⁸⁴ is ligated to the functional polypeptide is unlikely to be cleaved even in the presence of an excess amount of the protease described above and exhibits topical retention or sustained release because of the easy retention of the collagen-binding ability.

In addition, also in the process of the production in *E. coli* by the genetic engineering process, since the polypeptide sequence in human fibronectin from Asp ⁴⁸⁵ to Trp ⁵⁹⁹ has a sensitive site to proteases from *E. coli*, the hybrid polypeptide wherein the collagen-binding domain having the amino acid sequence which constitutes the polypeptide in human fibronectin from Ala ²⁶⁰ to Trp ⁵⁹⁹ is ligated to the functional polypeptide has constraints on the production conditions due to easy cleavage in *E. coli* or in the purification process. On the other hand, the hybrid polypeptide wherein the collagen-binding domain having the amino acid sequence which constitutes the polypeptide in human fibronectin from Ala ²⁶⁰ to Arg ⁴⁸⁴ is ligated to the functional polypeptide has the advantage that this hybrid polypeptide is not likely to be cleaved in *E. coli* or in the purification process.

It is possible to release the functional polypeptide without using any additional sequence by ligating the functional polypeptide on the carboxyl terminal side of the collagen-binding domain from FN and inserting an arbitrary amino acid sequence which can be cleaved by a specific sequence-cleaving protease (which exists for example in a human body) on the amino terminal side of the functional polypeptide. To be more specific, since many proteases cleave the carboxyl terminal of the recognition sequence, the amino terminal of the functional polypeptide has no unnecessary amino acid sequence after the cleavage with a protease if a protease recognition sequence exists on the amino terminal side of the functional polypeptide. Instead of newly inserting this protease recognition sequence, the carboxyl terminal side of the collagen-binding domain from FN may have a protease recognition sequence and a cleavage site. Therefore, insertion of artificial sequences can also be omitted by directly ligating the functional polypeptide to the carboxyl terminal. After the cleavage at this site, no unnecessary amino acid sequence remains in both of the collagen-binding domain from FN and the functional polypeptide. This method is important in the case where the functional polypeptide must be cleaved from the collagen-binding domain from FN to be released in a liquid phase. On the other hand, if another mode is possible in which the functional polypeptide exhibits its activity to cells by binding to a receptor through a solid phase as in matricrine and juxtacrine activities, it may be possible to exhibit the activity of the functional polypeptide by binding it to the receptor in the form of the hybrid polypeptide.

The collagen-binding hybrid polypeptide in which the FNCBD as described above is ligated to the functional polypeptide and which retains both the collagen-binding ability and the activity of the functional polypeptide enables stable retention of the functional polypeptide in the collagen matrix.

The collagen-binding hybrid polypeptide can have an amino acid or a polypeptide inserted as a spacer at the ligation site of the functional polypeptide and the collagen-binding domain.

In such a case, either the spacer, or the spacer and its adjacent sequence can preferably include a protease recognition sequence.

The protease recognition sequence is preferably a sequence recognizing enterokinase, blood coagulation factor Xa, thrombin, precision, kallikrein, Genenase I or renin. The protease recognition sequence is more preferably an enterokinase recognition sequence.

The enterokinase recognition sequence can play the role of a spacer which adjusts the distance between the FNCBD and the functional polypeptide and the enterokinase enables release of the functional polypeptide from the FNCBD. Enterokinase is a protease found in duodenal mucous membrane and pancreas of a higher animal. As a consequence, the enterokinase recognition sequence is recognized by the enterokinase in duodenal mucous membrane or pancreas to become cleaved, and the functional polypeptide is thereby released. Further, enterokinase is an enzyme which recognizes a sequence of very high specificity, DDDDK (Asp-Asp-Asp-Asp-Lys) to cleave C terminal side of K (Lys), and this sequence is necessary for the investigation of the mechanism as to how the functional polypeptide exhibits its activity in the collagen-binding hybrid polypeptide.

The protease-specific recognition sequence should be selected so that it cannot present in excess amounts under the condition under which the collagen-binding hybrid polypeptide is used. Insertion or retention of the protease-specific recognition sequence described above in the sequence of the collagen-binding hybrid polypeptide makes it difficult to retain the collagen-binding ability of the collagen binding hybrid polypeptide.

The collagen-binding ability of the hybrid polypeptide of the present invention is a collagen-binding ability dependent on the collagen-binding domain from FN, not the collagen-binding ability dependent on the functional polypeptide. Whether the collagen-binding ability is based on FN or the functional polypeptide may be confirmed by a competitive inhibition experiment in which a native FN or a corresponding functional polypeptide is used.

The term "collagen" or the "polypeptide from collagen" used herein designates a native collagen, an immature collagen, an atelocollagen, gelatin, or a polypeptide constituting such collagen forms.

An application of the hybrid polypeptide of the present invention is an agent which includes the collagen-binding hybrid polypeptide and which enables topical retention or sustained release of the functional polypeptide.

A biomaterial exhibiting a controlled activity is also provided. In this biomaterial, the functional polypeptide is gradually released from the biomaterial by the collagen decomposition or protease cleavage at the site of ligation between the functional polypeptide and the collagen-binding domain from FN or at a site in the vicinity of such ligation site.

The biomaterial as described above may be used to provide a method of promoting or inhibiting proliferation, differentiation, regeneration or biological substance production in a cell, a tissue or an organ.

There are also provided a collagen-binding hybrid polypeptide and a functional polypeptide / collagen composite matrix which can be used for sustained release, topical retention or targeting of the functional polypeptide. Biological tissues contain a substantial proportion of collagen, and collagen is present in substantially all tissues. Therefore, a functional polypeptide will be retained in the tissue at or near the site of administration when the functional polypeptide endowed with the collagen-binding ability. Then, the topical concentration of the functional polypeptide will be maintained at a high level and the efficacy of the functional polypeptide will be enhanced. Unfavorable side effects of the functional polypeptide caused by the diffusion of the functional polypeptide will be also prevented.

The present invention also provides a method of promoting cell proliferation wherein the collagen-binding hybrid polypeptide is used. To be more specific, the hybrid polypeptide of the present invention wherein the functional polypeptide is a cell growth factor that can bind to the collagen/gelatin matrix by utilizing its collagen-binding ability, and the cell growth factor will not flow away with the medium and thereby cell proliferation can be easily performed.

Also provided by the present invention are a gene coding for such collagen-binding hybrid polypeptide, a recombinant vector including such gene, a transformant having such gene (animal cell, insect cell, yeast, bacteria), and a bacterium, preferably *E. coli* having such gene. Also provided are a vector for gene therapy wherein the gene coding for the collagen-binding hybrid polypeptide is incorporated in the vector prepared by using a virus vector such as retrovirus, adenovirus, and adeno-associated vector, or a vector prepared by using liposome method. Introduction of the vector also makes it possible to provide a cell with a pharmaceutical function which contains the hybrid polypeptide or the gene.

A functional polypeptide/collagen composite matrix is provided by combining such hybrid polypeptide with the collagen, and this composite collagen matrix is quite useful as a novel biomaterial used in the medical field for tissue regeneration. To be more specific, the present invention provides a biomaterial comprising a functional polypeptide/collagen composite which simultaneously has a scaffold property and activity to enable the regeneration of artificial tissues and organs (blood vessel, nerve, ear, nose, finger, skin, gastrointestinal duct such as duodenum, stomach, heart, liver, pancreas, kidney, etc).

Next, the present invention is described in further detail by referring to preferred embodiments.

The cDNA and the protein primary structure of the human FN are described in EMBL DATA BANK and The EMBO Journal, vol. 4, No. 7, pages 1755-1759 (1985), respectively.

First, the gene corresponding to the amino acid sequence of the collagen-binding domain from Ala ²⁶⁰ to Arg ⁴⁸⁴ obtained by limited hydrolysis of the human FN with proteases (plasmin and trypsin) was cloned.

Reverse transcription reaction is conducted by using mRNA extracted from human cells, and the resulting cDNA is subjected to PCR (Polymerase Chain Reaction: Saiki et al., Science, vol. 230, pages 1350 to 1354 (1985)) to amplify the cDNA fragment corresponding to the human FN collagen-binding domain.

The sense primer used in the PCR has a restriction enzyme recognition sequence and the initiation codon sequence added on its 5' end, and the antisense primer has a restriction enzyme recognition sequence and antisense sequence of the termination codon added at its 5' end.

The FNCBD cDNA fragment is then inserted into the cloning vector, pBlueScript SK to construct plasmid pBS(FNCBD). After confirming the nucleotide sequence, the cDNA fragment is excised and incorporated in expression vector pTYB1 to construct plasmid pTYB1(FNCBD).

pTYB1(FMCBD) is a plasmid which expresses Ala ²⁶⁰-Arg ⁴⁸⁴ of human FN (225 amino acid residues), and introduction of the pTYB1(FNCBD) into *E. coli* enables the production of the collagen-binding domain.

A cDNA fragment from the plasmid pBS (FNCBD) is used for the cDNA of the FNCBD required in the present invention but according to the PCR primer design, the cDNA fragment has the initiation codon sequence added at the 5' end (corresponding Met is very often cleaved and removed after the translation), and a cloning site, for example, XhoI recognition sequence is introduced immediately before the termination codon added to the C terminal of the translation region. In this way, the cDNA of the FNCBD can be ligated with the cDNA of the functional polypeptide.

The polypeptide of the present invention is prepared by ligating the cDNA of the FNCBD and the cDNA of the functional polypeptide and expressing the resulting polypeptide by a genetic engineering process.

The functional polypeptide of the present invention is, for example, a collagen-binding hybrid polypeptide wherein a polypeptide of 225 amino acid residues corresponding to Ala ²⁶⁰ ₋ Arg ⁴⁸⁴ (corresponding to Ala ² - Arg ²²⁶ in SEQ ID NO. 4 of the Sequence Listing) of human FN obtained by limited hydrolysis with proteases (plasmin and trypsin) is ligated with human bFGF or human EGF represented by SEQ ID NO. 8 or SEQ ID NO. 12 in the Sequence Listing. In other words, the hybrid polypeptide is prepared through genetic engineering by ligating the cDNA of the FNCBD with the cDNA of the bFGF or EGF.

In the SEQ ID NO. 4 of the Sequence Listing, amino acid No. 1 represents Met translated from the initiation codon for expressing the human FNCBD by genetic engineering; amino acid Nos. 2 to 226 represent the amino acid sequence of the human FNCBD (corresponding to 260 to 484 of human FN); and amino acid Nos. 227 to 228 represent Leu and Glu encoded by the XhoI recognition sequence which is used for ligation with the cDNA of the functional polypeptide. It should be noted that, in the hybrid polypeptide, Glu will be removed upon the ligation of the XhoI recognition sequence and SalI recognition sequence.

In the SEQ ID NO. 8 of the Sequence Listing, amino acid No. 1 represents Asp encoded by the SalI recognition sequence used for ligation with the cDNA of FNCBD; amino acid Nos. 1 to 5 represent enterokinase recognition sequence (Asp-Asp-Asp-Asp-Lys ;DDDDK); and amino acid Nos. 6 to 159 represent the amino acid sequence of the human bFGF.

SEQ ID NO. 12 of the Sequence Listing, amino acid No. 1 represents Asp encoded by the SalI recognition sequence used for ligation with the cDNA of FNCBD; amino acid Nos. 1 to 5 represent enterokinase recognition sequence (DDDDK); and amino acid Nos. 6 to 58 represent the amino acid sequence of the human EGF.

It should be noted that, the numeral superscripts on the amino acids of the human FN correspond to the number of amino acid residues counted from the N terminal in the mature human FN described in EMBL DATA BANK. The polypeptide comprising 225 amino acid residues corresponding to Ala ²⁶⁰ - Arg ⁴⁸⁴ of human FN shown in SEQ ID NO. 4 (Ala ² - Arg ²²⁶) in the Sequence Listing has a different sequence from the amino acid sequence in EMBL DATA BANK by two amino acid residues, and this difference is not due to the PCR mutation.

The cDNA and the protein primary structure of the human bFGF are described in Kurokawa et al., FEBS Letter, vol. 213, No. 1, pages 189-194 (1987). The cDNA and the protein primary structure of the human EGF are described in Bell et al., Nucleic 20 Acids Research, vol. 14, No. 21, pages 8427-8446 (1986).

In the present invention, cDNAs of the human bFGF and the human EGF are amplified by PCR from the cDNA prepared by using human mRNA. The PCR of the respective cDNAs was conducted by using a sense primer having a restriction enzyme recognition sequence and a nucleotide sequence coding for a protease recognition sequence added at the 5' end and an antisense primer having a restriction enzyme recognition sequence added at the 5' end. These cDNA fragments were then inserted in pBluescript SK to prepare pBS(FGF) vector and pBS(EGF) vector, respectively.

The cDNA of the human FNCBD as described above is then ligated to the restriction enzyme recognition sequence on the 5' end of bFGF or EGF of the plasmid pBS(FGF) or pBS(EGF) to obtain plasmid pBS(FNCBD-FGF) or pBS(FNCBD-EGF) having the cDNA of the human FNCBD whose C terminal is ligated to the human bFGF or EGF. The fragment of the hybrid gene is excised from such plasmids and inserted in the expression vector pTYB1 to obtain the recombinant plasmid pTYB1(FNCBD-FGF) or pTYB1(FNCBD-EGF) which expresses the hybrid polypeptide represented by SEQ ID NO. 14 or SEQ ID NO. 16 in the Sequence Listing.

A nucleotide sequence derived from the PCR primer is present in the hybrid gene between the cDNA of the FNCBD and the cDNA of the bFGF or the EGF. Such sequence will be modified for the adjustment of the molecular distance between the bFGF or the EGF and the FNCBD and/or for the insertion of the protease recognition sequence such as the enterokinase recognition sequence or the like as the spacer peptide. The presence/absence and the type of the protease recognition sequence in the spacer peptide, sequence and length of the spacer peptide, and the like may be selected depending on the purpose of such spacer peptide insertion.

SEQ ID NO. 14 in the Sequence Listing is the amino acid sequence of the hybrid polypeptide of the human FNCBD and human bFGF. Amino acid No. 1 represents Met translated from the initiation codon; amino acid Nos. 2 to 226 represent the amino acid sequence of the human FNCBD; and amino acid Nos. 227 to 228 represent Leu and Asp coded by the nucleotide sequence produced by the ligation of the cDNA of the human FNCBD and the cDNA of the bFGF (ligation of the XhoI recognition sequence and the SalI recognition sequence); amino acid Nos. 228 to 232 represent enterokinase recognition sequence (DDDDK); and amino acid Nos. 233 to 385 represent the amino acid sequence of the human bFGF.

SEQ ID NO. 16 in the Sequence Listing is the amino acid sequence of the hybrid polypeptide of the human FNCBD and human EGF. Amino acid No. 1 represents Met translated from the initiation codon; amino acid Nos. 2 to 226 represent the amino acid sequence of the human FNCBD; and amino acid Nos. 227 to 228 represent Leu and Asp coded by the nucleotide sequence produced by the ligation of the cDNA of the human FNCBD and the cDNA of the EGF (ligation of the XhoI and SalI sites); amino acid Nos. 228 to 232 represent enterokinase recognition sequence (DDDDK); and amino acid Nos. 233 to 285 represent the amino acid sequence of the human EGF.

When the plasmids pTYB1(FNCBD), pTYB1(FNCBD-FGF) and pTYB1(FNCBD-EGF) constructed as described above are introduced in *E. coli.* respectively, and cultivated under appropriate conditions, the recombinant polypeptides are accumulated in the *E*. *coli.* Such expression may be confirmed by immunoblotting. To be more specific, the proteins from the whole cell of the recombinant *E. coli* are separated by SDS-polyacrylamide gel electrophoresis, and transferred to a nitrocellulose membrane. The bands of the recombinant polypeptides are then detected by using the monoclonal antibodies recognizing each of the FNCBD, the bFGF, and the EGF.

The polypeptide of the present invention is prepared, for example, as described below. The recombinant E. *coli* having the plasmid introduced therein is cultivated in a culture medium such as SB broth, and IPTG (isopropyl-β-D-thiogalactoside) is added to the culture medium to thereby induce the expression of the gene introduced in the cell. The cells are harvested after further cultivation, and the harvested cells are lysed by sonication. The cell lysate is centrifuged. The recombinant polypeptide in the form of inclusion body is included in the precipitate collected after the centrifugation, and the inclusion body is solubilized by denaturation with 8M urea .

Next, the solubilized inclusion body was dialyzed while decreasing the urea concentration stepwise, whereby the recombinant protein is renatured (refolding treatment). The thus prepared polypeptides of 30 kDa, 47 kDa, and 36 kDa are the embodiments of the FNCBD, the hybrid polypeptide of the FNCBD and the bFGF (FNCBD-FGF), and the hybrid polypeptide of the FNCBD and the EGF (FNCBD-EGF), respectively.

The collagen-binding ability of the thus prepared polypeptide may be examined by two processes. One is a batchwise process wherein the ability of the polypeptide to bind to gelatin (Gelatin Sepharose 4B, Amersham Pharmacia Biotech) is measured. A mixed suspension of the polypeptide and the Gelatin Sepharose 4B is put in a microtube of 1.5 ml and is stirred at 4ºC for 1 hour, and the suspension is subjected to centrifugation. The supernatant is collected, and the precipitate fraction is washed twice with 1M sodium chloride solution and the supernatant is collected. Elution of the polypeptide from gelatin is attempted by repeating the similar procedure with 1M urea, 2M urea, and 4M urea in this order. Finally, the precipitate is boiled in sodium dodecyl sulfate (SDS) buffer, and the supernatant is collected. The collected supernatant fractions are subjected to SDS-polyacrylamide gel electrophoresis (SDS-PAGE), and the binding and elution of the polypeptide to and from gelatin are determined by the presence and the absence of the corresponding band.

In the other process, ability of the FNCBD, the FNCBD-FGF. and the FNCBD-EGF to collagen of different forms, namely, gelatin, native collagen, and atelocollagen is measured. First, a 96-well plate is coated with gelatin, native collagen, and atelocollagen. The FNCBD, the FNCBD-FGF, and the FNCBD-EGF are dispensed, and the plate is incubated at 37ºC for about 1 hour, followed by washing and addition of anti-FNCBD monoclonal antibody. After further washing, reaction with a labeled secondary antibody is allowed to take place and the plate is washed. Enzyme substrate is then added for color development, and absorption in the well of the plate is measured for the collagen-binding ability.

Cell-growth promoting activity of the collagen-binding hybrid polypeptides may be evaluated by XTT method (Roehm et al., Journal Immunological Methods, vol. 142, pages 257 to 265 (1991)) or WST-1 method (Liu et al., Nature Medicine. vol. 1, pages 267 to 271 (1995)) wherein the dehydrogenase activity of the mitochondrial respiratory chain is measured for direct correlation to the number of living cells. To be more specific, mouse BALB/C3T3 cell or rat NRK49F cell is cultivated in a culture medium supplemented with each of the polypeptides described above for 3 or 4 days. XTT or WST-1 reagent is added and the cells are cultivated for further 3 to 6 hours. The culture medium is then measured for its absorption at a particular wavelength to evaluate the cell-growth promoting activity of the collagen-binding hybrid polypeptide.

The XTT method or the WST-1 method can also be used for the measurement of the cell-growth promoting activity of the collagen matrix combined with the collagen-binding hybrid polypeptide in the same manner as the above.

The hybrid polypeptide solution of different amount is added to a culture dish coated with collagen, and the solution is allowed to stand at 37ºC for 1 hour. When the polypeptide solution is removed and the dish is washed with PBS, there remains collagen matrix combined with the functional polypeptide. Balb/c3T3 mouse fibroblast or NRK49F rat fibroblast is cultivated on such matrix for 3 or 4 days, and for further 3 to 6 hours after adding the XTT or WST-1 reagent. The culture is then measured for its dehydrogenase activity as absorption at a particular wavelength to evaluate the cell-growth promoting activity of the collagen matrix combined with the functional polypeptide.

Further, the comparison between
the hybrid polypeptide wherein the collagen-binding domain having the amino acid sequence constituting the polypeptide in human fibronectin from Ala ²⁶⁰ to Arg ⁴⁸⁴ is ligated to the functional polypeptide and
the hybrid polypeptide wherein the collagen-binding domain having the amino acid sequence constituting the polypeptide in human fibronectin from Ala ²⁶⁰ to Trp ⁵⁹⁹ is ligated to the functional polypeptide
is possible for the functionality after binding to collagen.

The hybrid polypeptide solutions of different amounts are added to culture dishes coated with collagen, and the solutions are allowed to stand at 37ºC for 1 hour. After the polypeptide solutions are removed and the dishes are washed with PBS, there remain collagen matrices combined with the respective functional polypeptides. A protease-containing Hanks' buffer as described below or the like is dispensed in these matrices, which are allowed to stand at 37ºC for several hours. After the solution is removed and the dishes are washed, the activity of the collagen matrices combined with the functional polypeptides is measured.

This experiment shows that, in the presence of an excess amount of a protease selected from cathepsin G, cathepsin D, chymotrypsin, thermolysin, leucocyte elastase, chymase and the like, the hybrid polypeptide wherein the collagen-binding domain having the amino acid sequence constituting the polypeptide in human fibronectin from Ala ²⁶⁰ to Trp ⁵⁹⁹ is ligated to the functional polypeptide has difficulty in topical retention of the functional polypeptide whereas the hybrid polypeptide wherein the collagen-binding domain having the amino acid sequence constituting the polypeptide in human fibronectin from Ala ²⁶⁰ to Arg ⁴⁸⁴ is ligated to the functional polypeptide has the functional superiority in the topical retention or sustained release of the functional polypeptide.

Next, the present invention is described in further detail by referring to the Examples which by no means limit the scope of the invention.

### (Example 1)

Preparation of hybrid polypeptide of human FNCBD and human bFGF, and hybrid polypeptide of human FNCBD and human EGF

### (a) Cloning of the cDNA coding for human FNCBD

cDNA corresponding to the amino acid sequence of FNCBD obtained by limited hydrolysis of human FN with plasmin and trypsin was prepared by conducing reverse transcription of the mRNA extracted from human kidney cell with a primer (2), and the resulting cDNA was amplified with a pair of primers (1) and (2) through PCR (RT-PCR).

In the primer (1) represented by SEQ ID NO. 1 of the Sequence Listing,
nucleotide Nos. 1 to 2 are adjacent sequence added for ensuring KpnI digestion of the PCR product,
nucleotide Nos. 3 to 8 are KpnI recognition sequence for the cloning,
nucleotide Nos. 7 to 12 are NcoI recognition sequence,
nucleotide Nos. 13 to 14 are the sequence for adjusting the reading frame,
nucleotide Nos. 15 to 20 are NdeI recognition sequence,
nucleotide Nos. 18 to 20 are initiation codon sequence for the expression of the human FNCBD, and
nucleotide Nos. 21 to 49 are nucleotide sequence from the human FNCBD.

In the primer (2) represented by SEQ ID NO. 2 of the Sequence Listing,
nucleotide No. 1 is adjacent sequence added for ensuring EcoRI digestion of the PCR product,
nucleotide Nos. 2 to 7 are EcoRI recognition sequence for the cloning,
nucleotide Nos. 8 to 10 are antisense sequence of termination codon,
nucleotide Nos. 11 to 16 are XhoI recognition sequence for ligation with the cDNA of the bFGF, EGF, or other functional polypeptides, and
nucleotide Nos. 17 to 43 are antisense sequence from the cDNA of the human FNCBD.

RT-PCR was conducted with RNA LA PCR Kit (AMV) Ver. 1.1 (Takara Shuzo). First, reverse transcription of 0.8 µg of the total RNA was conducted at a reaction solution volume of 20 µl and at a temperature of 60ºC for 20 minutes, and the solution was further heated at 99ºC for 5 minutes. PCR using the resulting cDNA as the template was then carried out at a reaction solution volume of 100 µl. The solution was maintained at 94ºC for 1 minute, and the temperature cycle of 94ºC for 30 seconds, 63ºC for 1 minute, and 72ºC for 2 minutes was repeated 12 times. A 1/10 volume of the reaction solution was analyzed by agarose gel electrophoresis, and there was found a DNA fragment of about 770 bp. This fragment was of the size corresponding to that of the cDNA of the human FNCBD.

The thus amplified cDNA fragment was digested with KpnI and EcoRI, and ligated to a cloning vector pBluescript SK that had been digested with KpnI and EcoRI at 25ºC for 3 minutes (using the ligation kit Ver. 2 manufactured by Takara Shuzo).

Analysis of the nucleotide sequence revealed that a plasmid having incorporated therein the cDNA represented by SEQ ID NO. 3 in the Sequence Listing was obtained. This plasmid was designated pBS(FNCBD).

In SEQ ID NO. 3 of the Sequence Listing,
nucleotide Nos. 1 to 6 are KpnI recognition sequence for the cloning,
nucleotide Nos. 5 to 10 are NcoI recognition sequence,
nucleotide Nos. 11 to 12 are the sequence for adjusting the frame,
nucleotide Nos. 13 to 18 are NdeI recognition sequence,
nucleotide Nos. 16 to 18 are initiation codon sequence for the expression of human FNCBD,
nucleotide Nos. 19 to 693 are nucleotide sequence of the cDNA of the human FNCBD,
nucleotide Nos. 694 to 699 are XhoI recognition sequence for ligation to the cDNA of the bFGF, the EGF, or other functional polypeptides,
nucleotide Nos. 700 to 702 are termination codon, and
nucleotide Nos. 703 to 708 are EcoRI recognition sequence for the cloning.

It was found that the obtained cDNA sequence of the FNCBD was different from the one registered in EMBL DATA BANK by 5 nucleotides. However, it was confirmed that this difference was not caused by the point mutation in the PCR.

### (b) Cloning of the cDNA coding for human bFGF

cDNA of the human bFGF was prepared by conducting reverse transcription of the mRNA extracted from human kidney cell with a primer (4), and the resulting cDNA was amplified with a pair of primers (3) and (4) through PCR.

In the primer (3) represented by SEQ ID NO. 5 of the Sequence Listing,
nucleotide Nos. 1 to 2 are adjacent sequence added for ensuring SalI digestion of the PCR product,
nucleotide Nos. 3 to 8 are SalI recognition sequence for the cloning and for the ligation to the cDNA of the FNCBD,
nucleotide Nos. 6 to 20 are nucleotide sequence coding for enterokinase recognition sequence (DDDDK), and
nucleotide Nos. 21 to 40 are nucleotide sequence from the cDNA of the human bFGF.

In the primer (4) represented by SEQ ID NO. 6 of the Sequence Listing,
nucleotide No. 1 is adjacent sequence added for ensuring EcoRI digestion of the PCR product,
nucleotide Nos. 2 to 7 are EcoRI recognition sequence for the cloning,
nucleotide Nos. 8 to 10 are antisense sequence of termination codon, and
nucleotide Nos. 11 to 31 are antisense sequence of the cDNA of the human bFGF.

RT-PCR was conducted with RNA LA PCR Kit (AMV) Ver. 1.1 (Takara Shuzo). First, reverse transcription of 0.8 µg of the total RNA was conducted at a reaction solution volume of 20 µl and at a temperature of 60ºC for 20minutes, and the solution was further heated at 99ºC for 5 minutes. PCR reaction was then carried out in 100 µl of the reaction solution containing the cDNA. The solution was maintained at 94ºC for 2 minutes, and the temperature cycle of 94ºC for 30 seconds and 65ºC for 4 minutes was repeated 30 times. A 1/10 volume of the reaction solution was analyzed by agarose gel electrophoresis, and there was found a DNA fragment of about 450 bp. This fragment was of the size corresponding to that of the cDNA of the human bFGF.

The cDNA fragment was digested with SalI and EcoRI, and ligated to pBluescript SK that had been digested with SalI and EcoRI. A plasmid having incorporated therein the cDNA represented by SEQ ID NO. 7 in the Sequence Listing was obtained. This plasmid was designated pBS(FGF).

In SEQ ID NO. 7 of the Sequence Listing,
nucleotide Nos. 1 to 6 are SalI recognition sequence for the cloning and the ligation with the cDNA of the FNCBD,
nucleotide Nos. 4 to 18 are nucleotide sequence coding for enterokinase recognition sequence (DDDDK),
nucleotide Nos. 19 to 480 are nucleotide sequence of the cDNA of the human bFGF,
nucleotide Nos. 481 to 483 are termination codon, and
nucleotide Nos. 484 to 489 are EcoRI recognition sequence for the cloning.

It was found that the obtained nucleotide sequence of the human bFGF was different from the one registered in EMBL DATA BANK by 1 nucleotide. It was confirmed that this difference was caused by the point mutation in the PCR. However, this mutation was a silent point mutation with no change in the amino acid, and therefore, the sequence was used as it was.

### (c) Cloning of the cDNA coding for human EGF

cDNA of the human bFGF was prepared by conducting reverse transcription of the mRNA extracted from human kidney cell with a primer (6), and the resulting cDNA was amplified with a pair of primers (5) and (6) through PCR.

In the primer (5) represented by SEQ ID NO. 9 of the Sequence Listing,
nucleotide Nos. 1 to 2 are adjacent sequence added for ensuring SalI digestion of the PCR product,
nucleotide Nos. 3 to 8 are SalI recognition sequence for the cloning and for the ligation to the cDNA of the FNCBD,
nucleotide Nos. 6 to 20 are nucleotide sequence coding for enterokinase recognition sequence (DDDDK), and
nucleotide Nos. 21 to 44 are nucleotide sequence from the cDNA of the human EGF.

In the primer (6) represented by SEQ ID NO. 10 of the Sequence Listing,
nucleotide Nos. 1 to 6 are EcoRI recognition sequence for the cloning,
nucleotide Nos. 7 to 9 are antisense sequence of termination codon, and
nucleotide Nos. 10 to 30 are antisense sequence of the cDNA of the human EGF.

RT-PCR was conducted with TaKaRa RNA LA PCR Kit (AMV) Ver. 1.1 (Takara Shuzo). First, reverse transcription of 1.0 µg of the total RNA was conducted at a reaction solution volume of 20 µl and at a temperature of 60ºC for 30 minutes, and the solution was further heated at 99ºC for 5 minutes. PCR was then carried out at a reaction solution volume of 100 µl. The solution was maintained at 94ºC for 1 minute, and the temperature cycle of 94ºC for 30 seconds and 65ºC for 45 seconds was repeated 35 times.

A 1/10 volume of the reaction solution was analyzed by agarose gel electrophoresis, and there was found a DNA fragment of about 150 bp. This fragment was of the size corresponding to that of the cDNA of the human EGF.

The cDNA fragment was digested with SalI and EcoRI, and ligated to the pBluescript SK that had been digested with SalI and EcoRI.

A plasmid having incorporated therein the cDNA represented by SEQ ID NO. 11 in the Sequence Listing was obtained. This plasmid was designated pBS(EGF).

In SEQ ID NO. 11 of the Sequence Listing,
nucleotide Nos. 1 to 6 are SalI recognition sequence for the cloning and the ligation with the cDNA of the FNCBD,
nucleotide Nos. 4 to 18 are nucleotide sequence coding for enterokinase recognition sequence (DDDDK),
nucleotide Nos. 19 to 177 are nucleotide.sequence of the cDNA of the human EGF,
nucleotide Nos. 178 to 180 are termination codon, and
nucleotide Nos. 181 to 186 are EcoRI recognition sequence for the cloning.

It should be noted that the obtained nucleotide sequence of the human EGF was the same as the one registered in EMBL DATA BANK.

### (d) Preparation of the expression vector for human FNCBD

The plasmid pBS(FNCBD) obtained in the above (a) was treated with NdeI and EcoRI.

The inserted cDNA fragment of FNCBD was excised, and the fragment was ligated to the NdeI and EcoRI sites of the expression vector, pTYB1 (New England BioLab). The thus constructed plasmid was designated pTYB(FNCBD).

### (e) Preparation of expression vector for FNCBD/bFGF hybrid polypeptide

The plasmid pBS(FNCBD) obtained in the above (a) was digested with KpnI and XhoI. It should be noted that the plasmid was partially digested since XhoI-recognition sequence was present in the cDNA sequence of the FNCBD produced by RT-PCR of the renal RNA while XhoI-recognition sequence was absent in the sequence registered in EMBL DATA BANK.

The inserted cDNA fragment of the FNCBD was excised by the digestion as described above, and this fragment was ligated to KpnI and SalI sites of the plasmid pBS(FGF). The thus constructed plasmid had incorporated therein the DNA represented by SEQ ID NO. 13 of the Sequence Listing, and this plasmid was designated pBS(FNCBD-FGF).

In SEQ ID NO. 13 of the Sequence Listing,
nucleotide Nos. 1 to 6 are KpnI recognition sequence for the cloning,
nucleotide Nos. 5 to 10 are NcoI recognition sequence,
nucleotide Nos. 11 to 12 are the sequence for adjusting the reading frame,
nucleotide Nos. 13 to 18 are NdeI recognition sequence,
nucleotide Nos. 16 to 18 are initiation codon sequence,
nucleotide Nos. 19 to 693 are cDNA sequence of the human FNCBD.
nucleotide Nos. 694 to 699 are the nucleotide sequence formed by the ligation of the XhoI-recognition sequence and the Sali-recognition sequence,
nucleotide Nos. 697 to 711 are the nucleotide sequence coding for enterokinase recognition sequence (DDDDK),
nucleotide Nos. 712 to 1173 are cDNA sequence of the human bFGF,
nucleotide Nos. 1174 to 1176 are termination codon, and
nucleotide Nos. 1177 to 1182 are EcoRI recognition sequence for the cloning.

The DNA fragment of the hybrid gene that had been inserted in pBS(FNCBD-FGF) was excised with NdeI and EcoRI, and the fragment was ligated to NdeI and EcoRI sites of the expression vector, pTYB1.

The thus constructed plasmid was designated pTYBl(FNCBD-FGF).

### (f) Preparation of expression vector for FNCBD/EGF hybrid polypeptide

The DNA fragment of the FNCBD in the plasmid pBS(FNCBD) obtained in the above (a) was excised by treating (partially digesting) with KpnI and XhoI, and the fragment was ligated to KpnI and SalI sites of the plasmid pBS(EGF) obtained in the above (c).

The thus constructed plasmid had incorporated therein the DNA represented by SEQ ID NO. 15 of the Sequence Listing, and this plasmid was designated pBS(FNCBD-EGF).

In SEQ ID NO. 15 of the Sequence Listing,
nucleotide Nos. 1 to 6 are KpnI recognition sequence for the cloning,
nucleotide Nos. 5 to 10 are NcoI recognition sequence,
nucleotide Nos. 11 to 12 are the sequence for adjusting the expression frame,
nucleotide Nos. 13 to 18 are NdeI recognition sequence,
nucleotide Nos. 16 to 18 are initiation codon sequence,
nucleotide Nos. 19 to 693 are cDNA sequence of the human FNCBD,
nucleotide Nos. 694 to 699 are the nucleotide sequence formed by the ligation of the XhoI-recognition sequence and the SalI-recognition sequence,
nucleotide Nos. 697 to 711 are the nucleotide sequence coding for enterokinase recognition sequence (DDDDK),
nucleotide Nos. 712 to 870 are cDNA sequence of the EGF,
nucleotide Nos. 871 to 873 are termination codon, and
nucleotide Nos. 874 to 879 are EcoRI recognition sequence for the cloning.

The DNA fragment of the hybrid gene that had been inserted in pBS(FNCBD-EGF) was excised with NdeI and EcoRI, and the fragment was ligated to NdeI and EcoRI sites of the expression vector, pTYB1.

The thus constructed plasmid was designated pTYB1(FNCBD-EGF).

### (g) Confirmation of the expression of the FNCBD/bFGF hybrid polypeptide and FNCBD/EGF collagen-binding hybrid polypeptide

A strain of *E*. *coli,* ER2566 (NEW ENGLAND BioLabs) was transformed with pTYB1(FNCBD), pTYB1(FNCBD-FGF) and pTYB1(FNCBD-EGF), respectively.

The transformed *E*. *coli* was cultivated overnight in 2 ml of LB medium supplemented with 100 µg/ml ampicillin at 37ºC. 0.02 ml of this culture medium was inoculated in 2 ml of the SB medium supplemented with 100 µg/ml ampicillin.

After cultivating to a turbidity of 0.5, the culture medium was supplemented with IPTG (isopropyl-β-D-thiogalactoside) to a final concentration of 1mM and the cultivation was conducted at 37ºC for another 2 hours. The cells were then harvested.

The protein from the whole cell was subjected to SDS-PAGE (12% gel), and the gel was stained by Coomassie Brilliant Blue. Bands of 30 kDa, 47 kDa, and 36 kDa corresponding to the molecular weights of the FNCBD, the FNCBD/bFGF hybrid, and the FNCBD/EGF hybrid, respectively, were found from *E*. *coli* in the stained gel and the expression of the recombinant polypeptides were confirmed.

In the meanwhile, the gel after the SDS-PAGE was also transferred to a nitrocellulose membrane, and the membrane was reacted with a monoclonal antibody which specifically recognizes FNCBD (FNC4-4, Takara Shuzo), a monoclonal antibody which specifically recognizes bFGF (FGF-2 (C-18), Santa Cruz Biotechnology), and a monoclonal antibody which specifically recognizes EGF (Clone 10825.1 R&D Systems).

It was then confirmed that the anti-human FNCBD monoclonal antibody reacted with the polypeptide of 30 kDa, that the anti-human FNCBD monoclonal antibody and the anti-human bFGF monoclonal antibody reacted with the polypeptide of 47 kDa, and that the anti-human FNCBD monoclonal antibody and the anti-human EGF monoclonal antibody reacted with the polypeptide of 36 kDa.

These results indicated that the polypeptide of 30 kDa was the human FNCBD, the polypeptide of 47 kDa was the human FNCBD/human bFGF hybrid polypeptide (FNCBD-FGF), and the polypeptide of 36 kDa was the human FNCBD/human EGF hybrid polypeptide (FNCBD-EGF).

The *E. coli* ER2566 (NEW ENGLAND BioLabs) which had been transformed with the plasmid pTYB1(FNCBD), pTYB1(FNCBD-FGF), and pTYB1(FNCBD-EGF), respectively, as described above and in which the expression of the respective polypeptides were confirmed were designated ER2566[pTYB1(FNCBD)], ER2566[pTYB1(FNCBD-FGF)], and ER2566[pTYB1(FNCBD-EGF)], respectively.

### (h) Preparation of expressed polypeptide

*E. coli* obtained in the above (g) was preliminarily cultivated overnight in 2 ml of LB medium supplemented with 100 µg/ml ampicillin at 37ºC. 0.2 ml of this culture medium was inoculated in 2 ml of SB medium supplemented with 100 µg/ml ampicillin. After cultivating to a turbidity of 2, the culture medium was supplemented with IPTG to a final concentration of 10µM, the cultivation was conducted at 37ºC for further 16 hours and the cells were then harvested.

The thus collected cells were washed with sonication buffer (50mM Tris - HCl buffer, pH 8.0, 50mM sodium chloride, 1mM ethylenediaminetetraacetic acid (EDTA)) and centrifuged. The precipitate was suspended in the same buffer (2 ml) again, and the cells were lysed by repeating 6 cycles of sonication for 15 seconds with interval of 15 seconds under cooling with ice.

To this lysate was added TritonX-100 to a final concentration of 1%. and the lysate was centrifuged at 4ºC and at 5,000 rpm for 20 minutes. The precipitate obtained was further washed 3 times with an inclusion body-washing solution [0.5% TritonX-100, 1mM EDTA]. After centrifugation, the insoluble fraction was allowed to solubilize in 8M urea solution [8M urea; 50mM Tris-HCl buffer, pH 8.0; 1mM EDTA] at room temperature for 1 hour. Next, this solubilized solution was centrifuged at 4ºC and at 14,000 rpm for 20 minutes to collect the supernatant.

The thus obtained supernatant was dialyzed against 4M urea solution and 2M urea solution in this order, and finally, against sonication buffer or against phosphate buffer [10mM phosphate buffer, pH 8.0; 50mM sodium chloride] for use in the experiment of the cell-growth promoting activity.

After the dialysis, the supernatant was centrifuged at 4ºC and at 14,000 rpm for 20 minutes to prepare the water-soluble recombinant protein samples.

The thus obtained samples were subjected to SDS-PAGE, and it was then confirmed that each sample was a polypeptide as indicated by the band at the position of 30 kDa, 47 kDa, or 36 kDa corresponding to the molecular weight calculated from the amino acid sequence of the recombinant protein.

### (Example 2)

### <Evaluation of collagen-binding ability>

The FNCBD-bFGF and the FNCBD-EGF obtained in Example 1 were evaluated for their collagen-binding ability.

### Batch process

The ability of the FNCBD-bFGF and FNCBD-EGF to bind to gelatin (Gelatin Sepharose 4B, Amersham Pharmacia Biotech) was evaluated by a batch process.

The water-soluble recombinant protein samples used were about 50 µg/ml each of the FNCBD-bFGF and FNCBD-EGF which had been dialyzed against sonication buffer. To a 1.5 ml microtube was added 500 µl of the FNCBD-bFGF or FNCBD-EGF and 500 µl of the Gelatin Sepharose 4B (which had been washed twice with sonication buffer and prepared as 50% (vol/vol) suspension), and the mixture was stirred at 4ºC for 1 hour. The mixture was centrifuged at 5000 rpm for 30 seconds and the supernatant was collected. To the precipitate was added 500 µl of sonication buffer and the mixture was stirred at 4ºC for 1 hour. Then, the mixture was centrifuged at 5000 rpm for 30 seconds and the supernatant was collected. The precipitate was washed with Tris - HCl buffer, 1M sodium chloride solution and 2M sodium chloride solution in the same manner. Then, the precipitate was washed in the similar procedure with 1M urea, 2M urea, 4M urea and 8M urea in this order to elute protein bound to the Gelatin Sepharose 4B.

Finally, the suspension was heated to 90ºC in 500 µl of SDS buffer to elute protein bound to the Gelatin Sepharose 4B.

In the gel stained with Coomassie Brilliant Blue, the band of the FNCBD-bFGF or FNCBD-EGF around 47 kDa or 36 kDa calculated from the amino acid sequence was not detected in lane to which the supernatant after the reaction with gelatin was applied, which indicates that the FNCBD-bFGF and FNCBD-EGF bound to gelatin.

The band was not detected after washing with sonication buffer, Tris - HCl buffer, 1M sodium chloride solution and 2M sodium chloride solution, and it was then revealed that the FNCBD-bFGF and FNCBD-EGF are not eluted from gelatin by washing with these solutions.

The elutions with 1M urea, 2M urea, 4M urea and 8M urea were attempted in the similar procedure. As a result, the band was scarcely recognized in 1M urea and 2M urea and bands of 47 kDa and 36 kDa were detected in 4M urea and 8M urea, respectively. FNCBD-bFGF band and FNCBD-EGF band were detected also by the elution with SDS buffer at 90ºC.

In other words, elution of the FNCBD-bFGF and FNCBD-EGF were scarcely detected with 1 M urea and 2M urea, and substantially all of the FNCBD-bFGF and FNCBD-EGF were eluted with 4M urea, 8M urea and SDS buffer at 90ºC. Strong gelatin-binding ability of the FNCBD-bFGF and FNCBD-EGF. was thereby demonstrated. In the experiment below, polypeptide samples dialyzed against phosphate buffer after purification with the Gelatin Sepharose 4B were used.

### <ELISA process>

By the enzyme-linked immunosorbent assay (ELISA), binding ability of the FNCBD-bFGF and the FNCBD-EGF to bovine native collagen (I-AC, Koken), bovine atelocollagen (I-PC, Koken), and bovine serum albumin (BSA, Sigma) was evaluated.

First, 1 mg/ml solutions of native collagen, atelocollagen, and serum albumin were respectively dispensed in the wells of a flat-bottom 96-well ELISA plate at 200 µl/well, and the plate was allowed to stand overnight at 4ºC.

The wells were washed six times with PBS containing 0.05% Tween 20, and 100µl of the FNCBD-bFGF and the FNCBD-human EGF solutions diluted with PBS were respectively dispensed in the wells and the solution was allowed to stand at 37 °C for 1 hour.

The wells were washed three times with PBS containing 0.05% Tween 20, and 100 µl of anti-human FNCBD monoclonal antibody (Takara Shuzo) diluted 1:1000 was dispensed in the wells and allowed to stand at room temperature for 1 hour. The wells were washed three times with 0.05% Tween 20/PBS, and 100 µl of peroxidase-labelled anti-mouse immunoglobulin polyclonal antibody (Dako Japan) diluted 1:1000 was dispensed in the wells and allowed to stand at room temperature for 1 hour. After washing the wells 6 times with 0.05% Tween 20/PBS, 0.1M citrate buffer, pH 4.7 containing 1 mg/ml o-phenylenediamine and 0.03% hydrogen peroxide was dispensed in the wells and allowed to stand for 10 minutes. The reaction was then terminated by addition of 50 µl of 4N sulfuric acid and absorption at 492 - 690 nm was measured.

As a result of the reaction of the water-soluble FNCBD-bFGF and FNCBD-EGF with native collagen, atelocollagen, or serum albumin, the absorption in the case of native collagen and atelocollagen was clearly higher than that in the case of using serum albumin. As described above, the binding ability of the FNCBD-bFGF and FNCBD-EGF to native collagen and atelocollagen showed markedly higher values than the binding ability of the FNCBD-bFGF and FNCBD-EGF to serum albumin.

Absorption in ELISA is considered to increase in correlation with the binding ability. Since absorption after the reactions of the FNCBD-bFGF and FNCBD-EGF with the immobilized native collagen and atelocollagen is clearly higher than that after the reactions of the FNCBD-bFGF and FNCBD-EGF with the immobilized serum albumin whose binding may be regarded as a non-specific binding, these reactions are regarded to be specific reactions. This means that the polypeptide produced in the present invention by genetic engineering is a molecule wherein the collagen-binding domain has been fused with the functional polypeptide while the collagen-binding ability inherent in the FNCBD is not lost. In summary, it was possible to impart the fibronectin-derived collagen-binding ability to the functional polypeptide by producing a hybrid polypeptide.

### (Example 3)

### <Cell-growth promoting activity >

The FNCBD, the FNCBD-bFGF, and the FNCBD-EGF which exhibited the collagen-binding ability in Example 2 above were also evaluated for their cell-growth promoting activity.

Balb/c3T3 cells (FNCBD-bFGF) or NRK49F cells (FNCBD-EGF) were suspended in Iscove's Modified Dulbecco's Medium containing 2% fetal bovine serum (2%FBS-IMDM), and the suspension was dispensed in 24-well plate at 1 × 10⁴ cells/well. The cells were cultivated at 37ºC for 7 hours in the presence of 5% CO₂. The water-soluble FNCBD, FNCBD-bEGF, or FNCBD-EGF was then added to the cell culture, and the cultivation was continued at 37ºC for 3 days. WST-1 method was used for the measurement of the cell-growth promoting activity. the reaction solution was transferred to a 96-well microtiter plate, and absorption at 450 nm-690 nm was measured with a microplate reader to evaluate the cell-growth promoting activity.

Each of the FNCBD-bEGF and the FNCBD-EGF clearly exhibited a concentration-dependent cell-growth promoting activity to the Balb/c3T3 cell or NRK49F cell compared with the FNCBD. Therefore, the cell-growth promoting activity of the human bFGF and EGF is not considered to be lost by the fusion of the FNDBD with the bFGF or EGF.

### (Example 4)

### <Cell-growth promoting activity after binding to collagen>

The cell-growth promoting activity of the collagen-binding hybrid polypeptide bound to collagen was studied.

The FNCBD-bEGF and FNCBD-EGF were used as examples of the collagen-binding hybrid polypeptide whose collagen-binding ability and cell-growth promoting activity had been confirmed. The FNCBD-bEGF and FNCBD-EGF were evaluated for their cell-growth promoting activity after their binding to collagen (FNCBD-bEGF or FNCBD-EGF /collagen composite). First, 1 mg/ml solution of atelocollagen in HCl, pH 3.0 was dispensed in the wells of a 24-well culture plate at 500 µl/well. and the solution was allowed to stand overnight at 4ºC. The solution was discarded, and the wells were washed four times with PBS-0.05% Tween 20, twice with PBS, and once with serum-free DMEM. Next, 250 µl of the FNCBD-bEGF, FNCBD-EGF or FNCBD solution serially diluted with serum-free DMEM was dispensed in each well, and the solution was allowed to stand at 37°C for 2 hours. The solution was discarded, and the wells were washed twice with PBS-0.05% Tween 20, 6 times with PBS, and once with Hanks' solution. Then, Balb/c3T3 cells (FNCBD-bFGF) or NRK49F cells (FNCBD-EGF) were suspended in 2% FBS-DMEM, and the suspension was dispensed in the wells of a 24-well plate at 10⁴ cells/0.5 ml/well. Thereafter, the cells were cultivated in the presence of 5% CO₂ at 37°C for 3 days. WST-1 method was used for the measurement of the cell-growth promoting activity. The reaction solution was transferred to a 96-well microtiter plate, and absorption at 450 nm - 690 nm was measured to evaluate the cell-growth promoting activity.

Each of the FNCBD-bEGF and the FNCBD-EGF clearly exhibited a concentration-dependent cell-growth promoting activity to the Balb/c3T3 cell or NRK49F cell compared with the FNCBD. Therefore, it has been revealed that the FNCBD-bEGF or the FNCBD-EGF is a hybrid polypeptide wherein the fibronectin-derived collagen-binding domain is ligated to the functional polypeptide, bFGF or EGF and this hybrid polypeptide exhibits the cell-growth promoting activity after the binding to collagen by retaining or gradually releasing its binding to the collagen.

In addition, the collagen having the FNCBD-bEGF or FNCBD-EGF bound thereto is an example of the biomaterial which includes both of the collagen-binding hybrid polypeptide and the polypeptide from the collagen (functional polypeptide/collagen composite). The method of cultivating the cells as described above is applied to an agent for enabling topical retention or sustained release of a functional polypeptide using the functional polypeptide/collagen composite.

### (Example 5)

### <Comparison of functionality based on distinction between collagen-binding domains>

The cell-growth promoting activity after binding to collagen was compared between
the hybrid polypeptide of the present invention wherein the collagen-binding domain having the amino acid sequence constituting the polypeptide in human fibronectin from Ala ²⁶⁰ to Arg ⁴⁸⁴ (corresponding to Ala ² - Arg ²²⁶ of SEQ ID NO. 4 in the Sequence Listing) is ligated to the EGF (hereinafter referred to as "FN484-EGF") and
the hybrid polypeptide wherein the collagen-binding domain having the amino acid sequence constituting the polypeptide in human fibronectin from Ala ²⁶⁰ to Trp ⁵⁹⁹ is ligated to the EGF (hereinafter referred to as "FN599-EGF").

After binding to collagen each of water-soluble FN484-EGF and FN599-EGF whose collagen-binding ability and cell-growth promoting activity had been confirmed (FN484-EGF or FN599-EGF/collagen composite), their cell-growth promoting activity was evaluated. First, 1 mg/ml solution of type I atelocollagen in HCl (pH 3.0) was dispensed in the wells of a 24-well culture plate at 500 µl/well. and the solution was allowed to stand overnight at 4ºC. The solution was discarded, and the wells were washed four times with PBS-0.05% Tween 20, twice with PBS, and once with serum-free DMEM. Next, 250 µl of the FN484-EGF or FN599-EGF solution serially diluted with serum-free DMEM was dispensed in each well, and the solution was allowed to stand at 37ºC for 2 hours. The solution was discarded, and the wells were washed twice with PBS-0.05% Tween 20, 6 times with PBS, and once with Hanks' buffer.

After washing, cathepsin G-containing Hanks' buffer whose final concentration is 10 ng/ml was dispensed at 500 µl/well, and the solution was allowed to stand at 37ºC for 2 hours. The solution was discarded, and the wells were washed twice with PBS-0.05% Tween 20, 6 times with PBS, and once with Hanks' buffer.

Next, NRK49F cells were suspended in 2% FBS-DMEM, and the suspension was dispensed in the wells of a 24-well plate at 10⁴ cells/0.5 ml/well. Thereafter, the cells were cultivated in the presence of 5% CO₂ at 37°C for 3 days. WST-1 method was used for the measurement of the cell-growth promoting activity. The reaction solution was transferred to a 96-well microtiter plate, and absorption at 450 nm - 690 nm was measured to evaluate the cell-growth promoting activity.

The FN484-EGF clearly exhibited a concentration-dependent cell-growth promoting activity to the NRK49F cells compared with the FN599-EGF. Therefore, it has been revealed that the FN484-EGF is a collagen-binding hybrid polypeptide wherein the hybrid polypeptide exposed to cathepsin G after binding to collagen still exhibits the cell-growth promoting activity.

This result shows that the FN484-EGF has a functional superiority in its capability to exhibit the EGF activity even in living tissues in which cathepsin G exists.

### (Example 6)

### <Topical retention of FN484-EGF bound to collagen and its capability to maintain cell-growth promoting activity in the presence of leukocyte elastase>

The FN484-EGF and the FN599-EGF whose collagen-binding ability and cell-growth promoting activity had been confirmed in Examples 2 to 4 were evaluated for their topical retention and capability to maintain the cell-growth promoting activity after binding to collagen in the presence of leukocyte elastase. The functional superiority was compared between the FN484-EGF and the FN599-EGF.

First, 1 mg/ml solution of type I atelocollagen in HCl (pH 3.0) was dispensed in the wells of a 24-well culture plate at 500 µl/well, and the solution was allowed to stand overnight at 4°C. The solution was discarded, and the wells were washed twice with PBS-0.05% Tween 20, 4 times with PBS, and once with serum-free DMEM. Next, 250 µl of the FN484-EGF or FN599-EGF solution serially diluted with serum-free DMEM (0 nM, 0.04 nM, 0.2 nM, 1 nM, 5 nM, 25 nM) was dispensed in each well, and the solution was allowed to stand at 37°C for 2 hours. The solution was discarded, and the wells were washed twice with PBS-0.05% Tween 20, 6 times with PBS, and once with Hanks' buffer.

After washing, leukocyte elastase-containing Hanks' buffer whose final concentration is 0.1 unit/ml was dispensed at 500 µl/well, and the solution was allowed to stand at 37°C for 2 hours. The solution was discarded, and the wells were washed twice with PBS-0.05% Tween 20, 4 times with PBS, and once with Hanks' buffer.

Next, NRK49F cells were suspended in 2% FBS-DMEM, and the suspension was dispensed in the wells of the 24-well plate at 10⁴ cells/0.5 ml/well. Thereafter, the cells were cultivated in the presence of 5% CO₂ at 37°C for 3 days. WST-1 method was used for the measurement of the cell-growth promoting activity. The reaction solution of 100 µl was transferred to each well of a 96-well microtiter plate, and absorption at 450 nm - 690 nm was measured with a microplate reader to evaluate the cell-growth promoting activity.

According to the growth test of the NRK49F cells exposed to leukocyte elastase, the FN599-EGF could not promote the cell growth while being topically retained, whereas the FN484-EGF exhibited a concentration-dependent cell-growth promoting activity. The difference of the cell-growth promoting activity between the FN484-EGF and the FN599-EGF bound to collagen was more significant after exposure to leukocyte elastase than in the presence of cathepsin G. Therefore, it has been revealed that the FN484-EGF is a collagen-binding hybrid polypeptide which has a function of exhibiting the cell-growth promoting activity even if it is exposed to leukocyte elastase after having been bound to collagen.

This result shows that the FN484-EGF has a functional superiority in that the FN484-EGF bound to collagen exhibits the EGF activity by being topically retained therein or by being thereafter gradually released therefrom and hence is effective for tissue regeneration.

In addition, in the hybrid polypeptide wherein the collagen-binding domain having the amino acid sequence which constitutes the polypeptide in human fibronectin from Ala ²⁶⁰ to Arg ⁴⁸⁴ is ligated to a functional polypeptide such as cytokine (including a cell growth factor) (this polypeptide being synonymous with a physiologically active peptide), the FN484-functional polypeptide bound to collagen exhibits its function (physiological activity) even in living tissues in which leukocyte elastase exists, by being topically retained therein or by being thereafter gradually released therefrom. It can be said that the FN484-functional polypeptide has a functional superiority in the drug effect such as promoting tissue regeneration.

Even if the collagen-binding domain having the amino acid sequence which constitutes the polypeptide in human fibronectin from Ala ²⁶⁰ to Arg ⁴⁸⁴ is replaced by the collagen-binding domain having the amino acid sequence which constitutes the polypeptide 260 - 484 in FN from species other than human, it is clear that the latter has the same function as the former.

### Industrial Applicability

The hybrid polypeptide of the present invention has both the collagen-binding ability and the activity of the functional polypeptide by having a specific collagen-binding domain of FN and can also retain these activities consistently even in living tissues in which an excess amount of an enzyme exists.

There are provided a hybrid polypeptide and a biomaterial in which the hybrid polypeptide is combined with a collagen-derived polypeptide.

FREE TEXT OF SEQUENCE LISTING

SEQ ID NO: 1

Description of Artificial Sequence: PCR Sense Primer for

Human Fibronectin Collagen-Binding Domain

SEQ ID NO: 2

Description of Artificial Sequence: PCR Antisense Primer

for Human Fibronectin Collagen-Binding Domain

SEQ ID NO: 3

Description of Artificial Sequence: Nucleotide Sequence of

DNA Coding for Human Fibronectin Collagen-Binding Domain

SEQ ID NO: 4

Description of Artificial Sequence: Amino Acid Sequence of

Human Fibronectin Collagen-Binding Domain

(2) ... (226):/note = "Human Fibronectin Collagen-Binding

Domain"

SEQ ID NO: 5

Description of Artificial Sequence: PCR Sense Primer for

Basic Fibroblast Growth Factor

SEQ ID NO: 6

Description of Artificial Sequence: PCR Antisense Primer

for Basic Fibroblast Growth Factor

SEQ ID NO: 7

Description of Artificial Sequence: Nucleotide Sequence of
DNA Coding for Basic Fibroblast Growth Factor with
Enterokinase Recognition Sequence
SEQ ID NO: 8
Description of Artificial Sequence: Amino Acid Sequence of
Basic Fibroblast Growth Factor with Enterokinase
Recognition Sequence
(1) ... (5):/note = "Enterokinase Recognition Sequence"
(6) ... (159):/note = "Basic Fibroblast Growth Factor"
SEQ ID NO: 9
Description of Artificial Sequence: PCR Sense Primer for
Epidermal Growth Factor
SEQ ID NO: 10
Description of Artificial Sequence: PCR Antisense Primer
for Epidermal Growth Factor
SEQ ID NO: 11
Description of Artificial Sequence: Nucleotide Sequence of
DNA Coding for Epidermal Growth Factor with Enterokinase
Recognition Sequence
SEQ ID NO: 12
Description of Artificial Sequence: Amino Acid Sequence of
Epidermal Growth Factor with Enterokinase Recognition
Sequence
(1) .. (5):/note = "Enterokinase Recognition Sequence"

(6) .. (58):/note = "Epidermal Growth Factor"
SEQ ID NO: 13
Description of Artificial Sequence:  Nucleotide Sequence of
DNA Coding for Collagen-Binding Basic Fibroblast Growth
Factor
SEQ ID NO: 14
Description of Artificial Sequence:  Amino Acid Sequence of
Collagen-Binding Basic Fibroblast Growth Factor
(2) .. (226):/note = "Human Fibronectin Collagen-Binding
Domain"
(228) .. (232):/note = "Enterokinase Recognition Sequence"
(233) .. (386):/note = "Basic Fibroblast Growth Factor"
SEQ ID NO: 15
Description of Artificial Sequence:  Nucleotide Sequence of
DNA Coding for Collagen-Binding Epidermal Growth Factor
SEQ ID NO: 16
Description of Artificial Sequence:  Amino Acid Sequence of
Collagen-Binding Epidermal Growth Factor
(2) .. (226):/note = "Human Fibronectin Collagen-Binding
Domain"
(228) .. (232):/note = "Enterokinase Recognition Sequence"
(233) .. (285):/note = "Epidermal Growth Factor"

## Claims

1. A hybrid polypeptide wherein a collagen-binding domain having an amino acid sequence which constitutes the polypeptide in human fibronectin from Ala ²⁶⁰ to Arg ⁴⁸⁴; an amino acid sequence which is homologous to said amino acid sequence; or an amino acid sequence in which one or more amino acid residues are deleted, substituted, inserted or added is ligated to a functional polypeptide.

2. A hybrid polypeptide according to claim 1, wherein said functional polypeptide is ligated to the carboxyl terminal of said collagen-binding domain.

3. A biomaterial containing a functional polypeptide/collagen composite in which a hybrid polypeptide according to claim 1 or 2 is combined with a collagen-derived polypeptide.

4. A gene coding for a hybrid polypeptide according to claim 1 or 2.

5. A transformant containing a gene according to claim 4.


Sequence Listing

<110> Terumo Corporation

<120> Collagen-binding hybrid polypetide

<130> TE200123

<160> 16

<170> PatentIn Ver. 2.1

<210> 1

<211> 49

<212> DNA

<213> Artificial Sequence

<220>

<223> Description of Artificial Sequence:PCR Sense

Primer for Human Fibronectin Collagen-Binding

Domain

<400> 1

gaggtaccat ggtacatatg gcagctgttt accaaccgca gcctcaccc 49

<210> 2

<211> 43

<212> DNA

<213> Artificial Sequence

<220>

<223> Description of Artificial Sequence: PCR Antisense

Primer for Human Fibronectin Collagen-Binding

Domain

<400> 2

ggaattctca ctcgagtcga agctgcgagt aggcaatgca tgt 43

<210> 3

<211> 708

<212> DNA

<213> Artificial Sequence

<220>

<223> Description of Artificial Sequence: Human Fibronectin

Collagen-binding Domain

<220>

<221> CDS

<222> (16)..(693)

<220>

<221> conflict

<222> (109)

<220>

<221> conflict

<222> (206)

<220>

<221> conflict

<222> (270)

<220>

<221> conflict

<222> (374)

<220>

<221> conflict

<222> (681)

<400> 3

ggtaccatgg tacat atg gca gct gtt tac caa ccg cag cct cac ccc cag 51

Met Ala Ala Val Tyr Gln Pro Gln Pro His Pro Gln

1               5                   10

cct cct ccc tat ggc cac tgt gtc aca gac agt ggt gtg gtc tac tct 99

Pro Pro Pro Tyr Gly His Cys Val Thr Asp Ser Gly Val Val Tyr Ser

        15                  20                  25

gtg ggg atg cag tgg ctg aag aca caa gga aat aag caa atg ctt tgc 147

Val Gly Met Gln Trp Leu Lys Thr Gln Gly Asn Lys Gln Met Leu Cys

    30                  35                  40

acg tgc ctg ggc aac gga gtc agc tgc caa gag aca gct gta acc cag 195

Thr Cys Leu Gly Asn Gly Val Ser Cys Gln Glu Thr Ala Val Thr Gln
45                  50                  55                  60
act tac ggt ggc aac tca aat gga gag cca tgt gtc tta cca ttc acc 243
Thr Tyr Gly Gly Asn Ser Asn Gly Glu Pro Cys Val Leu Pro Phe Thr
                65                  70                  75
tac aat ggc agg acg ttc tac tcc tgc acc aca gaa ggg cga cag gac 291
Tyr Asn Gly Arg Thr Phe Tyr Ser Cys Thr Thr Glu Gly Arg Gln Asp
            80                  85                  90
gga cat ctt tgg tgc agc aca act tcg aat tat gag cag gac cag aaa 339
Gly His Leu Trp Cys Ser Thr Thr Ser Asn Tyr Glu Gln Asp Gln Lys
        95                  100                 105
tac tct ttc tgc aca gac cac act gtt ttg gtt cag act cga gga gga 387
Tyr Ser Phe Cys Thr Asp His Thr Val Leu Val Gln Thr Arg Gly Gly
    110                 115                 120
aat tcc aat ggt gcc ttg tgc cac ttc ccc ttc cta tac aac aac cac 435
Asn Ser Asn Gly Ala Leu Cys His Phe Pro Phe Leu Tyr Asn Asn His
125                 130                 135                 140
aat tac act gat tgc act tct gag ggc aga aga gac aac atg aag tgg 483
Asn Tyr Thr Asp Cys Thr Ser Glu Gly Arg Arg Asp Asn Met Lys Trp
                145                 150                 155
tgt ggg acc aca cag aac tat gat gcc gac cag aag ttt ggg ttc tgc 531
Cys Gly Thr Thr Gln Asn Tyr Asp Ala Asp Gln Lys Phe Gly Phe Cys

            160                 165                 170

ccc atg gct gcc cac gag gaa atc tgc aca acc aat gaa ggg gtc atg 579

Pro Met Ala Ala His Glu Glu Ile Cys Thr Thr Asn Glu Gly Val Met

        175                 180                 185

tac cgc att gga gat cag tgg gat aag cag cat gac atg ggt cac atg 627

Tyr Arg Ile Gly Asp Gln Trp Asp Lys Gln His Asp Met Gly His Met

    190                 195                 200

atg agg tgc acg tgt gtt ggg aat ggt cgt ggg gaa tgg aca tgc att 675

Met Arg Cys Thr Cys Val Gly Asn Gly Arg Gly Glu Trp Thr Cys Ile

205                 210                 215                 220

gcc tac tcg cag ctt cga ctc gag tgagaattc 708

Ala Tyr Ser Gln Leu Arg Leu Glu

                225

<210> 4

<211> 228

<212> PRT

<213> Artificial Sequence

<223> Description of Artificial Sequence: Human Fibronectin

Collagen-binding Domain

<400> 4

Met Ala Ala Val Tyr Gln Pro Gln Pro His Pro Gln Pro Pro Pro Tyr

1                   5                   10                  15

Gly His Cys Val Thr Asp Ser Gly Val Val Tyr Ser Val Gly Met Gln

                20                  25                  30

Trp Leu Lys Thr Gln Gly Asn Lys Gln Met Leu Cys Thr Cys Leu Gly

            35                  40                  45

Asn Gly Val Ser Cys Gln Glu Thr Ala Val Thr Gln Thr Tyr Gly Gly

        50                  55                  60

Asn Ser Asn Gly Glu Pro Cys Val Leu Pro Phe Thr Tyr Asn Gly Arg

65                  70                  75                  80

Thr Phe Tyr Ser Cys Thr Thr Glu Gly Arg Gln Asp Gly His Leu Trp

                85                  90                  95

Cys Ser Thr Thr Ser Asn Tyr Glu Gln Asp Gln Lys Tyr Ser Phe Cys

            100                 105                 110

Thr Asp His Thr Val Leu Val Gln Thr Arg Gly Gly Asn Ser Asn Gly

        115                 120                 125

Ala Leu Cys His Phe Pro Phe Leu Tyr Asn Asn His Asn Tyr Thr Asp

    130                 135                 140

Cys Thr Ser Glu Gly Arg Arg Asp Asn Met Lys Trp Cys Gly Thr Thr

145                 150                 155                 160

Gln Asn Tyr Asp Ala Asp Gln Lys Phe Gly Phe Cys Pro Met Ala Ala

                165                 170                 175

His Glu Glu Ile Cys Thr Thr Asn Glu Gly Val Met Tyr Arg Ile Gly

            180                 185                 190

Asp Gln Trp Asp Lys Gln His Asp Met Gly His Met Met Arg Cys Thr

        195                 200                 205

Cys Val Gly Asn Gly Arg Gly Glu Trp Thr Cys Ile Ala Tyr Ser Gln

    210                 215                 220

Leu Arg Leu Glu

225

<210> 5

<211> 40

<212> DNA

<213> Artificial Sequence

<220>

<223> Description of Artificial Sequence: PCR Sense

Primer for Human Basic Fibroblast Growth Factor

<400> 5

gagtcgacga cgatgataag gcagccggga gcatcaccac 40

<210> 6

<211> 31

<212> DNA

<213> Artificial Sequence

<220>

<223> Description of Artificial Sequence: PCR Antisense

Primer for Human Basic Fibroblast Growth Factor

<400> 6

ggaattctca gctcttagca gacattggaa g 31

<210> 7

<211> 489

<212> DNA

<213> Artificial Sequence

<220>

<223> Description of Artificial Sequence: Human Basic

Fibroblast Growth Factor with Enterokinase

Recognition Sequence

<220>

<221> CDS

<222> (4)..(480)

<220>

<221> mutation

<222> (228)

<223> /note="mutation caused by polymerase chain

reaction"

<400> 7

gtc gac gac gat gat aag gca gcc ggg agc atc acc acg ctg ccc gcc 48

Asp Asp Asp Asp Lys Ala Ala Gly Ser Ile Thr Thr Leu Pro Ala

1               5                   10                  15

ttg ccc gag gat ggc ggc agc ggc gcc ttc ccg ccc ggc cac ttc aag 96

Leu Pro Glu Asp Gly Gly Ser Gly Ala Phe Pro Pro Gly His Phe Lys

                20                  25                  30

gac ccc aag cgg ctg tac tgc aaa aac ggg ggc ttc ttc ctg cgc atc 144

Asp Pro Lys Arg Leu Tyr Cys Lys Asn Gly Gly Phe Phe Leu Arg Ile

            35                  40                  45

cac ccc gac ggc cga gtt gac ggg gtc cgg gag aag agc gac cct cac 192

His Pro Asp Gly Arg Val Asp Gly Val Arg Glu Lys Ser Asp Pro His

        50                  55                  60

atc aag cta caa ctt caa gca gaa gag aga gga gtc gtg tct atc aaa 240

Ile Lys Leu Gln Leu Gln Ala Glu Glu Arg Gly Val Val Ser Ile Lys

    65                  70                  75

gga gtg tgt gct aac cgt tac ctg gct atg aag gaa gat gga aga tta 288

Gly Val Cys Ala Asn Arg Tyr Leu Ala Met Lys Glu Asp Gly Arg Leu

80                  85                  90                  95

ctg gct tct aaa tgt gtt acg gat gag tgt ttc ttt ttt gaa cga ttg 336

Leu Ala Ser Lys Cys Val Thr Asp Glu Cys Phe Phe Phe Glu Arg Leu

                100                 105                 110

gaa tct aat aac tac aat act tac cgg tca agg aaa tac acc agt tgg    384

Glu Ser Asn Asn Tyr Asn Thr Tyr Arg Ser Arg Lys Tyr Thr Ser Trp

            115                 120                 125

tat gtg gca ctg aaa cga act ggg cag tat aaa ctt gga tcc aaa aca    432

Tyr Val Ala Leu Lys Arg Thr Gly Gln Tyr Lys Leu Gly Ser Lys Thr

        130                 135                 140

gga cct ggg cag aaa gct ata ctt ttt ctt cca atg tct gct aag agc    480

Gly Pro Gly Gln Lys Ala Ile Leu Phe Leu Pro Met Ser Ala Lys Ser

    145                 150                 155

tgagaattc                                                          489

<210> 8

<211> 159

<212> PRT

<213> Artificial Sequence

<223> Description of Artificial Sequence: Human Basic

      Fibroblast Growth Factor with Enterokinase

      Recognition Sequence

<400> 8

Asp Asp Asp Asp Lys Ala Ala Gly Ser Ile Thr Thr Leu Pro Ala Leu

  1               5                  10                  15

Pro Glu Asp Gly Gly Ser Gly Ala Phe Pro Pro Gly His Phe Lys Asp

            20                  25                  30

Pro Lys Arg Leu Tyr Cys Lys Asn Gly Gly Phe Phe Leu Arg Ile His

        35                  40                  45

Pro Asp Gly Arg Val Asp Gly Val Arg Glu Lys Ser Asp Pro His Ile

    50                  55                  60

Lys Leu Gln Leu Gln Ala Glu Glu Arg Gly Val Val Ser Ile Lys Gly

65                  70                  75                  80

Val Cys Ala Asn Arg Tyr Leu Ala Met Lys Glu Asp Gly Arg Leu Leu

                85                  90                  95

Ala Ser Lys Cys Val Thr Asp Glu Cys Phe Phe Phe Glu Arg Leu Glu

            100                 105                 110

Ser Asn Asn Tyr Asn Thr Tyr Arg Ser Arg Lys Tyr Thr Ser Trp Tyr

        115                 120                 125

Val Ala Leu Lys Arg Thr Gly Gln Tyr Lys Leu Gly Ser Lys Thr Gly

    130                 135                 140

Pro Gly Gln Lys Ala Ile Leu Phe Leu Pro Met Ser Ala Lys Ser

145                 150                 155

<210> 9

<211> 44

<212> DNA

<213> Artificial Sequence

<220>

<223> Description of Artificial Sequence: PCR Sense

      Primer for Human Epidermal Growth Factor

<400> 9

gtgtcgacga cgatgataag aatagtgact ctgaatgtcc cctg                    44

<210> 10

<211> 30

<212> DNA

<213> Artificial Sequence

<220>

<223> Description of Artificial Sequence: PCR Antisense

      Primer for Human Epidermal Growth Factor

<400> 10

gaattcttag cgcagttccc accacttcag                                    30

<210> 11

<211> 186

<212> DNA

<213> Artificial Sequence

<220>

<223> Description of Artificial Sequence: Human Epidermal

Growth Factor with Enterokinase Recognition Sequence

<220>

<221> CDS

<222> (4)..(177)

<400> 11

gtc gac gac gat gat aag aat agt gac tct gaa tgt ccc ctg tcc cac 48

Asp Asp Asp Asp Lys Asn Ser Asp Ser Glu Cys Pro Leu Ser His

1               5                   10                  15

gat ggg tac tgc ctc cat gat ggt gtg tgc atg tat att gaa gca ttg 96

Asp Gly Tyr Cys Leu His Asp Gly Val Cys Met Tyr Ile Glu Ala Leu

                20                  25                  30

gac aag tat gca tgc aac tgt gtt gtt ggc tac atc ggg gag cga tgt 144

Asp Lys Tyr Ala Cys Asn Cys Val Val Gly Tyr Ile Gly Glu Arg Cys

            35                  40                  45

cag tac cga gac ctg aag tgg tgg gaa ctg cgc taagaattc 186

Gln Tyr Arg Asp Leu Lys Trp Trp Glu Leu Arg

        50                  55

<210> 12

<211> 58

<212> PRT

<213> Artificial Sequence

<223> Description of Artificial Sequence: Human Epidermal

Growth Factor with Enterokinase Recognition Sequence

<400> 12

Asp Asp Asp Asp Lys Asn Ser Asp Ser Glu Cys Pro Leu Ser His Asp

1               5                   10                  15

Gly Tyr Cys Leu His Asp Gly Val Cys Met Tyr Ile Glu Ala Leu Asp

            20                  25                  30

Lys Tyr Ala Cys Asn Cys Val Val Gly Tyr Ile Gly Glu Arg Cys Gln

        35                  40                  45

Tyr Arg Asp Leu Lys Trp Trp Glu Leu Arg

    50                  55

<210> 13

<211> 1182

<212> DNA

<213> Artificial Sequence

<220>

<223> Description of Artificial Sequence: Collagen-binding Basic

Fibroblast Growth Factor

<220>

<221> CDS

<222> (16)..(1173)

<400> 13

ggtaccatgg tacat atg gca gct gtt tac caa ccg cag cct cac ccc cag 51

Met Ala Ala Val Tyr Gln Pro Gln Pro His Pro Gln

1               5                   10

cct cct ccc tat ggc cac tgt gtc aca gac agt ggt gtg gtc tac tct 99

Pro Pro Pro Tyr Gly His Cys Val Thr Asp Ser Gly Val Val Tyr Ser

        15                  20                  25

gtg ggg atg cag tgg ctg aag aca caa gga aat aag caa atg ctt tgc 147

Val Gly Met Gln Trp Leu Lys Thr Gln Gly Asn Lys Gln Met Leu Cys

    30                  35                  40

acg tgc ctg ggc aac gga gtc agc tgc caa gag aca gct gta acc cag 195

Thr Cys Leu Gly Asn Gly Val Ser Cys Gln Glu Thr Ala Val Thr Gln

45                  50                  55                  60

act tac ggt ggc aac tca aat gga gag cca tgt gtc tta cca ttc acc 243

Thr Tyr Gly Gly Asn Ser Asn Gly Glu Pro Cys Val Leu Pro Phe Thr

                65                  70                  75

tac aat ggc agg acg ttc tac tcc tgc acc aca gaa ggg cga cag gac 291

Tyr Asn Gly Arg Thr Phe Tyr Ser Cys Thr Thr Glu Gly Arg Gln Asp

            80                  85                  90

gga cat ctt tgg tgc agc aca act tcg aat tat gag cag gac cag aaa 339

Gly His Leu Trp Cys Ser Thr Thr Ser Asn Tyr Glu Gln Asp Gln Lys

        95                  100                 105

tac tct ttc tgc aca gac cac act gtt ttg gtt cag act cga gga gga 387
Tyr Ser Phe Cys Thr Asp His Thr Val Leu Val Gln Thr Arg Gly Gly
    110                 115                 120
aat tcc aat ggt gcc ttg tgc cac ttc ccc ttc cta tac aac aac cac 435
Asn Ser Asn Gly Ala Leu Cys His Phe Pro Phe Leu Tyr Asn Asn His
125                 130                 135                 140
aat tac act gat tgc act tct gag ggc aga aga gac aac atg aag tgg 483
Asn Tyr Thr Asp Cys Thr Ser Glu Gly Arg Arg Asp Asn Met Lys Trp
                145                 150                 155
tgt ggg acc aca cag aac tat gat gcc gac cag aag ttt ggg ttc tgc 531
Cys Gly Thr Thr Gln Asn Tyr Asp Ala Asp Gln Lys Phe Gly Phe Cys
            160                 165                 170
ccc atg gct gcc cac gag gaa atc tgc aca acc aat gaa ggg gtc atg 579
Pro Met Ala Ala His Glu Glu Ile Cys Thr Thr Asn Glu Gly Val Met
        175                 180                 185
tac cgc att gga gat cag tgg gat aag cag cat gac atg ggt cac atg 627
Tyr Arg Ile Gly Asp Gln Trp Asp Lys Gln His Asp Met Gly His Met
    190                 195                 200
atg agg tgc acg tgt gtt ggg aat ggt cgt ggg gaa tgg aca tgc att 675
Met Arg Cys Thr Cys Val Gly Asn Gly Arg Gly Glu Trp Thr Cys Ile
205                 210                 215                 220
gcc tac tcg cag ctt cga ctc gac gac gat gat aag gca gcc ggg agc 723

Ala Tyr Ser Gln Leu Arg Leu Asp Asp Asp Asp Lys Ala Ala Gly Ser

                225                 230                 235

atc acc acg ctg ccc gcc ttg ccc gag gat ggc ggc agc ggc gcc ttc 771

Ile Thr Thr Leu Pro Ala Leu Pro Glu Asp Gly Gly Ser Gly Ala Phe

            240                 245                 250

ccg ccc ggc cac ttc aag gac ccc aag cgg ctg tac tgc aaa aac ggg 819

Pro Pro Gly His Phe Lys Asp Pro Lys Arg Leu Tyr Cys Lys Asn Gly

        255                 260                 265

ggc ttc ttc ctg cgc atc cac ccc gac ggc cga gtt gac ggg gtc cgg 867

Gly Phe Phe Leu Arg Ile His Pro Asp Gly Arg Val Asp Gly Val Arg

    270                 275                 280

gag aag agc gac cct cac atc aag cta caa ctt caa gca gaa gag aga 915

Glu Lys Ser Asp Pro His Ile Lys Leu Gln Leu Gln Ala Glu Glu Arg

285                 290                 295                 300

gga gtc gtg tct atc aaa gga gtg tgt gct aac cgt tac ctg gct atg 963

Gly Val Val Ser Ile Lys Gly Val Cys Ala Asn Arg Tyr Leu Ala Met

                305                 310                 315

aag gaa gat gga aga tta ctg gct tct aaa tgt gtt acg gat gag tgt 1011

Lys Glu Asp Gly Arg Leu Leu Ala Ser Lys Cys Val Thr Asp Glu Cys

            320                 325                 330

ttc ttt ttt gaa cga ttg gaa tct aat aac tac aat act tac cgg tca 1059

Phe Phe Phe Glu Arg Leu Glu Ser Asn Asn Tyr Asn Thr Tyr Arg Ser

        335                 340                 345

agg aaa tac acc agt tgg tat gtg gca ctg aaa cga act ggg cag tat 1107

Arg Lys Tyr Thr Ser Trp Tyr Val Ala Leu Lys Arg Thr Gly Gln Tyr

    350                 355                 360

aaa ctt gga tcc aaa aca gga cct ggg cag aaa gct ata ctt ttt ctt 1155

Lys Leu Gly Ser Lys Thr Gly Pro Gly Gln Lys Ala Ile Leu Phe Leu

365                 370                 375                 380

cca atg tct gct aag agc tgagaattc 1182

Pro Met Ser Ala Lys Ser

                385

<210> 14

<211> 386

<212> PRT

<213> Artificial Sequence

<223> Description of Artificial

Sequence: Collagen-binding Basic Fibroblast Growth

Factor

<400> 14

Met Ala Ala Val Tyr Gln Pro Gln Pro His Pro Gln Pro Pro Pro Tyr

1               5                   10                  15

Gly His Cys Val Thr Asp Ser Gly Val Val Tyr Ser Val Gly Met Gln

            20                  25                  30
Trp Leu Lys Thr Gln Gly Asn Lys Gln Met Leu Cys Thr Cys Leu Gly
        35                  40                  45
Asn Gly Val Ser Cys Gln Glu Thr Ala Val Thr Gln Thr Tyr Gly Gly
    50                  55                  60
Asn Ser Asn Gly Glu Pro Cys Val Leu Pro Phe Thr Tyr Asn Gly Arg
65                  70                  75                  80
Thr Phe Tyr Ser Cys Thr Thr Glu Gly Arg Gln Asp Gly His Leu Trp
                85                  90                  95
Cys Ser Thr Thr Ser Asn Tyr Glu Gln Asp Gln Lys Tyr Ser Phe Cys
            100                 105                 110
Thr Asp His Thr Val Leu Val Gln Thr Arg Gly Gly Asn Ser Asn Gly
        115                 120                 125
Ala Leu Cys His Phe Pro Phe Leu Tyr Asn Asn His Asn Tyr Thr Asp
    130                 135                 140
Cys Thr Ser Glu Gly Arg Arg Asp Asn Met Lys Trp Cys Gly Thr Thr
145                 150                 155                 160
Gln Asn Tyr Asp Ala Asp Gln Lys Phe Gly Phe Cys Pro Met Ala Ala
                165                 170                 175
His Glu Glu Ile Cys Thr Thr Asn Glu Gly Val Met Tyr Arg Ile Gly
            180                 185                 190
Asp Gln Trp Asp Lys Gln His Asp Met Gly His Met Met Arg Cys Thr

        195                 200                 205
Cys Val Gly Asn Gly Arg Gly Glu Trp Thr Cys Ile Ala Tyr Ser Gln
    210                 215                 220
Leu Arg Leu Asp Asp Asp Asp Lys Ala Ala Gly Ser Ile Thr Thr Leu
225                 230                 235                 240
Pro Ala Leu Pro Glu Asp Gly Gly Ser Gly Ala Phe Pro Pro Gly His
                245                 250                 255
Phe Lys Asp Pro Lys Arg Leu Tyr Cys Lys Asn Gly Gly Phe Phe Leu
            260                 265                 270
Arg Ile His Pro Asp Gly Arg Val Asp Gly Val Arg Glu Lys Ser Asp
        275                 280                 285
Pro His Ile Lys Leu Gln Leu Gln Ala Glu Glu Arg Gly Val Val Ser
    290                 295                 300
Ile Lys Gly Val Cys Ala Asn Arg Tyr Leu Ala Met Lys Glu Asp Gly
305                 310                 315                 320
Arg Leu Leu Ala Ser Lys Cys Val Thr Asp Glu Cys Phe Phe Phe Glu
                325                 330                 335
Arg Leu Glu Ser Asn Asn Tyr Asn Thr Tyr Arg Ser Arg Lys Tyr Thr
            340                 345                 350
Ser Trp Tyr Val Ala Leu Lys Arg Thr Gly Gln Tyr Lys Leu Gly Ser
        355                 360                 365
Lys Thr Gly Pro Gly Gln Lys Ala Ile Leu Phe Leu Pro Met Ser Ala

    370                 375                 380

Lys Ser

385

<210> 15

<211> 879

<212> DNA

<213> Artificial Sequence

<220>

<223> Description of Artificial Sequence: Collagen-binding

Epidermal Growth Factor

<220>

<221> CDS

<222> (16)..(870)

<400> 15

ggtaccatgg tacat atg gca gct gtt tac caa ccg cag cct cac ccc cag 51

Met Ala Ala Val Tyr Gln Pro Gln Pro His Pro Gln

1               5                   10

cct cct ccc tat ggc cac tgt gtc aca gac agt ggt gtg gtc tac tct 99

Pro Pro Pro Tyr Gly His Cys Val Thr Asp Ser Gly Val Val Tyr Ser

        15                  20                  25

gtg ggg atg cag tgg ctg aag aca caa gga aat aag caa atg ctt tgc 147

Val Gly Met Gln Trp Leu Lys Thr Gln Gly Asn Lys Gln Met Leu Cys
    30                  35                  40
acg tgc ctg ggc aac gga gtc agc tgc caa gag aca gct gta acc cag 195
Thr Cys Leu Gly Asn Gly Val Ser Cys Gln Glu Thr Ala Val Thr Gln
45                  50                  55                  60
act tac ggt ggc aac tca aat gga gag cca tgt gtc tta cca ttc acc 243
Thr Tyr Gly Gly Asn Ser Asn Gly Glu Pro Cys Val Leu Pro Phe Thr
                65                  70                  75
tac aat ggc agg acg ttc tac tcc tgc acc aca gaa ggg cga cag gac 291
Tyr Asn Gly Arg Thr Phe Tyr Ser Cys Thr Thr Glu Gly Arg Gln Asp
            80                  85                  90
gga cat ctt tgg tgc agc aca act tcg aat tat gag cag gac cag aaa 339
Gly His Leu Trp Cys Ser Thr Thr Ser Asn Tyr Glu Gln Asp Gln Lys
        95                  100                 105
tac tct ttc tgc aca gac cac act gtt ttg gtt cag act cga gga gga 387
Tyr Ser Phe Cys Thr Asp His Thr Val Leu Val Gln Thr Arg Gly Gly
    110                 115                 120
aat tcc aat ggt gcc ttg tgc cac ttc ccc ttc cta tac aac aac cac 435
Asn Ser Asn Gly Ala Leu Cys His Phe Pro Phe Leu Tyr Asn Asn His
125                 130                 135                 140
aat tac act gat tgc act tct gag ggc aga aga gac aac atg aag tgg 483
Asn Tyr Thr Asp Cys Thr Ser Glu Gly Arg Arg Asp Asn Met Lys Trp

                145                 150                 155

tgt ggg acc aca cag aac tat gat gcc gac cag aag ttt ggg ttc tgc 531

Cys Gly Thr Thr Gln Asn Tyr Asp Ala Asp Gln Lys Phe Gly Phe Cys

            160                 165                 170

ccc atg gct gcc cac gag gaa atc tgc aca acc aat gaa ggg gtc atg 579

Pro Met Ala Ala His Glu Glu Ile Cys Thr Thr Asn Glu Gly Val Met

        175                 180                 185

tac cgc att gga gat cag tgg gat aag cag cat gac atg ggt cac atg 627

Tyr Arg Ile Gly Asp Gln Trp Asp Lys Gln His Asp Met Gly His Met

    190                 195                 200

atg agg tgc acg tgt gtt ggg aat ggt cgt ggg gaa tgg aca tgc att 675

Met Arg Cys Thr Cys Val Gly Asn Gly Arg Gly Glu Trp Thr Cys Ile

205                 210                 215                 220

gcc tac tcg cag ctt cga ctc gac gac gat gat aag aat agt gac tct 723

Ala Tyr Ser Gln Leu Arg Leu Asp Asp Asp Asp Lys Asn Ser Asp Ser

                225                 230                 235

gaa tgt ccc ctg tcc cac gat ggg tac tgc ctc cat gat ggt gtg tgc 771

Glu Cys Pro Leu Ser His Asp Gly Tyr Cys Leu His Asp Gly Val Cys

            240                 245                 250

atg tat att gaa gca ttg gac aag tat gca tgc aac tgt gtt gtt ggc 819

Met Tyr Ile Glu Ala Leu Asp Lys Tyr Ala Cys Asn Cys Val Val Gly

        255                 260                 265

tac atc ggg gag cga tgt cag tac cga gac ctg aag tgg tgg gaa ctg    867
Tyr Ile Gly Glu Arg Cys Gln Tyr Arg Asp Leu Lys Trp Trp Glu Leu
    270                 275                 280
cgc taagaattc                                                      879
Arg
285

<210> 16
<211> 285
<212> PRT
<213> Artificial Sequence
<223> Description of Artificial Sequence: Collagen-binding
Epidermal Growth Factor
<400> 16
Met Ala Ala Val Tyr Gln Pro Gln Pro His Pro Gln Pro Pro Pro Tyr
  1               5                  10                  15
Gly His Cys Val Thr Asp Ser Gly Val Val Tyr Ser Val Gly Met Gln
             20                  25                  30
Trp Leu Lys Thr Gln Gly Asn Lys Gln Met Leu Cys Thr Cys Leu Gly
         35                  40                  45
Asn Gly Val Ser Cys Gln Glu Thr Ala Val Thr Gln Thr Tyr Gly Gly
     50                  55                  60

Asn Ser Asn Gly Glu Pro Cys Val Leu Pro Phe Thr Tyr Asn Gly Arg
65                  70                  75                  80
Thr Phe Tyr Ser Cys Thr Thr Glu Gly Arg Gln Asp Gly His Leu Trp
                85                  90                  95
Cys Ser Thr Thr Ser Asn Tyr Glu Gln Asp Gln Lys Tyr Ser Phe Cys
            100                 105                 110
Thr Asp His Thr Val Leu Val Gln Thr Arg Gly Gly Asn Ser Asn Gly
        115                 120                 125
Ala Leu Cys His Phe Pro Phe Leu Tyr Asn Asn His Asn Tyr Thr Asp
    130                 135                 140
Cys Thr Ser Glu Gly Arg Arg Asp Asn Met Lys Trp Cys Gly Thr Thr
145                 150                 155                 160
Gln Asn Tyr Asp Ala Asp Gln Lys Phe Gly Phe Cys Pro Met Ala Ala
                165                 170                 175
His Glu Glu Ile Cys Thr Thr Asn Glu Gly Val Met Tyr Arg Ile Gly
            180                 185                 190
Asp Gln Trp Asp Lys Gln His Asp Met Gly His Met Met Arg Cys Thr
        195                 200                 205
Cys Val Gly Asn Gly Arg Gly Glu Trp Thr Cys Ile Ala Tyr Ser Gln
    210                 215                 220
Leu Arg Leu Asp Asp Asp Asp Lys Asn Ser Asp Ser Glu Cys Pro Leu
225                 230                 235                 240

Ser His Asp Gly Tyr Cys Leu His Asp Gly Val Cys Met Tyr Ile Glu

                245                 250                 255

Ala Leu Asp Lys Tyr Ala Cys Asn Cys Val Val Gly Tyr Ile Gly Glu

            260                 265                 270

Arg Cys Gln Tyr Arg Asp Leu Lys Trp Trp Glu Leu Arg

        275                 280                 285